(19) Europäisches Patentamt
European Patent Office
Office européen des brevets

(11) Publication number: **0 535 227 A1**

(12) **EUROPEAN PATENT APPLICATION published in accordance with Art. 158(3) EPC**

(21) Application number: **91909700.6**

(22) Date of filing: **28.05.91**

(86) International application number: **PCT/JP91/00712**

(87) International publication number: **WO 91/18882 (12.12.91 91/28)**

(51) Int. Cl.[5]: **C07D 213/77**, C07D 277/42, C07D 401/12, C07D 413/12, C07D 417/12

(30) Priority: **29.05.90 JP 137121/90**
**19.04.91 JP 115293/91**

(43) Date of publication of application: **07.04.93 Bulletin 93/14**

(84) Designated Contracting States: **AT BE CH DE DK ES FR GB GR IT LI LU NL SE**

(71) Applicant: **NIPPON SODA CO., LTD.**
**2-1, Ohtemachi 2-chome**
**Chiyoda-ku, Tokyo 100(JP)**

(72) Inventor: **ISHIMITSU, Keiichi, Odawara Research Center,**
**Nippon Soda Co., Ltd. 345, Aza Yanagimachi Takada, Odawara-shi Kanagawa 250-02(JP)**
Inventor: **SUZUKI, Junji, Odawara Research Center,**
**Nippon Soda Co., Ltd. 345, Aza Yanagimachi Takada, Odawara-shi Kanagawa 250-02(JP)**
Inventor: **KISHIMOTO, Takashi, Odawara Research Center,**
**Nippon Soda Co., Ltd. 345, Aza Yanagimachi Takada, Odawara-shi Kanagawa 250-02(JP)**
Inventor: **OHISHI, Haruhito, Odawara Research Center,**
**Nippon Soda Co., Ltd. 345, Aza Yanagimachi Takada, Odawara-shi Kanagawa 250-02(JP)**
Inventor: **YAMADA, Tomio, Odawara Research Center,**
**Nippon Soda Co., Ltd. 345, Aza Yanagimachi Takada, Odawara-shi Kanagawa 250-02(JP)**
Inventor: **HATANO, Renpei, Odawara Research Center,**
**Nippon Soda Co., Ltd. 345, Aza Yanagimachi Takada, Odawara-shi Kanagawa 250-02(JP)**
Inventor: **TAKAKUSA, Nobuo, Odawara Research Center,**
**Nippon Soda Co., Ltd. 345, Aza Yanagimachi Takada, Odawara-shi Kanagawa 250-02(JP)**

(74) Representative: **de Bruijn, Leendert C. (NL) et al**
**Nederlandsch Octrooibureau**
**Scheveningseweg 82 P.O. Box 29720**
**NL-2505 KZ Den Haag (NL)**

(54) **N-SUBSTITUTED HETEROCYCLIC AMIDINE DERIVATIVE.**

(57) A compound of general formula (I) or its salt, having a potent insecticidal activity wherein $R_1$ represents optionally substituted aromatic nitrogenous heterocycle; X represents optionally substituted alkylene or alkylidene; $R_2$ represents hydrogen, optionally substituted alkyl, alkenyl, alkynyl, cycloalkyl, cycloalkenyl, aryl or $-Y-R_5$ (wherein Y represents O, $S(O)_n$,-CO- or $-CO_2-$, n represents 0, 1 or 2, and $R_5$ represents hydrogen, optionally substituted alkyl, alkenyl, alkynyl, cycloalkyl, cycloalkenyl or aryl); $R_3$ represents hydrogen, optionally substituted alkyl, alkenyl, alkynyl, cycloalkyl, cycloalkenyl, heterocycle, $-OR_6$, $-SR_6$ (wherein $R_6$ represents hydrogen, optionally substituted alkyl, alkenyl, alkynyl, cycloalkyl, cycloalkenyl or aryl), or (a) wherein $R_7$ represents hydrogen, optionally substituted alkyl, alkenyl, alkynyl, cycloalkyl, cycloalkenyl or aryl, and $R_8$ represents hydrogen, optionally substitued alkyl, alkenyl, alkynyl, cycloalkyl, cycloalkenyl, aryl or $-Z-R_9$ (wherein

EP 0 535 227 A1

Z represents O, S(O)k, -CO- or $-CO_2-$, It represents 0, 1 or 2, and $R_9$ represents hydrogen, optionally substituted alkyl, alkenyl, alkynyl, cycloalkyl, cycloalkenyl or aryl), or alternatively $R_7$ and $R_8$ and/or $R_2$ and $R_3$ may be combined together with optionally a heteroatom to form a ring; and $R_4$ represents optionally substituted nitrogenous heterocycle.

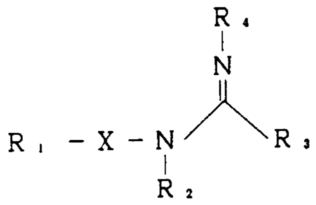

〔 I 〕

Technical Fields:

This invention is concerning to N-substituted heterocyclic amidine derivatives, the processes for preparing them and the insecticidal compositions containing the derivatives as an active ingredient.

Background Technology:

Many kinds of insecticide, for example, organophosphates such as parathion, malathion and others and carbamates such as carbaryl, methomyl and others, have been developed and practically applied as the results of research and development on applied entomology for many years. These insecticides have acted very important roles to improve agricultural productivity, on the other hand, recently some of the insecticides have been facing to problems of regulation for applying them, because of their residual, accumulative and polluting deficiencies in the environment or of expression of resistant strain of target pest insects with these insecticides according to their frequent exposure for long period. Therefore, the development of a new pesticide which is outstandingly effective to either the resistant or the other various kinds of pest insects and more safely applicable has been expected.

Purpose of the invention is to provide such a pesticide which is able to be industrially synthesized with profitable conditions, reliable effectiveness and more safe.

Disclosure of The Invention:

This invention is concerning to compounds which are represented by general formula [I], their salts, the processes for preparing them and the insecticidal compositions containing them.

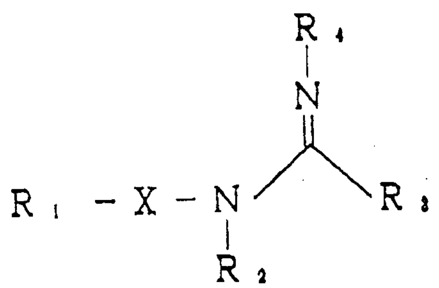

( I )

[wherein, $R_1$ is aromatic heterocycles containing nitrogen allowed to be substituted; X is alkylene or alkylidene allowed to be substituted in each case;

$R_2$ is hydrogen, alkyl, alkenyl, alkynyl, cycloalkyl, cycloalkenyl, aryl allowed to be substituted in each case or $-Y-R_5$, wherein Y is O, S(O)n, -CO- or $-CO_2-$, wherein n denotes 1 or 2 and $R_5$ is hydrogen, alkyl, alkenyl, alkynyl, cycloalkyl cycloalkenyl or aryl allowed to be substituted in each case;

$R_3$ is hydrogen, alkyl, alkenyl, alkynyl, cycloalkyl, cycloalkenyl, heterocycles allowed to be substituted. $OR_6$, $SR_6$ (wherein, $R_6$ is hydrogen, alkyl, alkenyl, alkynyl, cycloalkyl, cycloalkenyl or aryl allowed to be substituted in each case) or $-NR_7R_8$, (wherein $R_7$ is hydrogen, alkyl, alkenyl, alkynyl, cycloalkyl, aryl cycloalkenyl or aryl allowed to be substituted in each case, $R_6$ is hydrogen, alkyl, alkenyl, alkynyl, cycloalkyl, cycloalkenyl, aryl allowed to be substituted in each case) or $Z-R_9$ wherein Z is O, $S(O)_k$ -CO or $CO_2$ in which k denotes zero, 1 or 2 and $R_9$ is hydrogen, alkyl, alkenyl, alkynyl, cycloalkyl, cycloalkenyl or aryl allowed to be substituted in each case), $R_7$ and $R_8$, and /or, $R_2$ and $R_3$ are allowed to form a ring by binding each other which may contain further hetero atom; and $R_4$ is heterocycles containing nitrogen allowed to be substituted].

The compounds in the invention are prepared by following processes;

(1) Process 1:

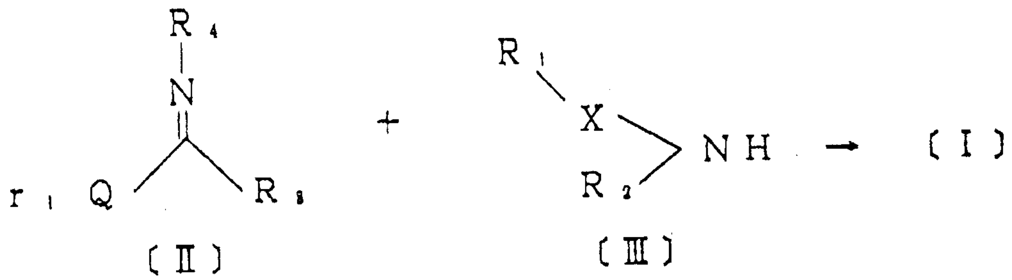

Wherein $r_1$ is lower alkyl; Q is oxygen or sulfar; and X, $R_1$-$R_4$ are same as above.

The reaction is desirably carried out under refluxing condition in inert solvent desirably alcohols such as ethanol or aromatic hydrocarbons such as toluene or xylene, but may be proceeded at room temperature in more polar solvents such as DMF.

(2) Process 2: In the case $R_3$ is $NR_7R_8$

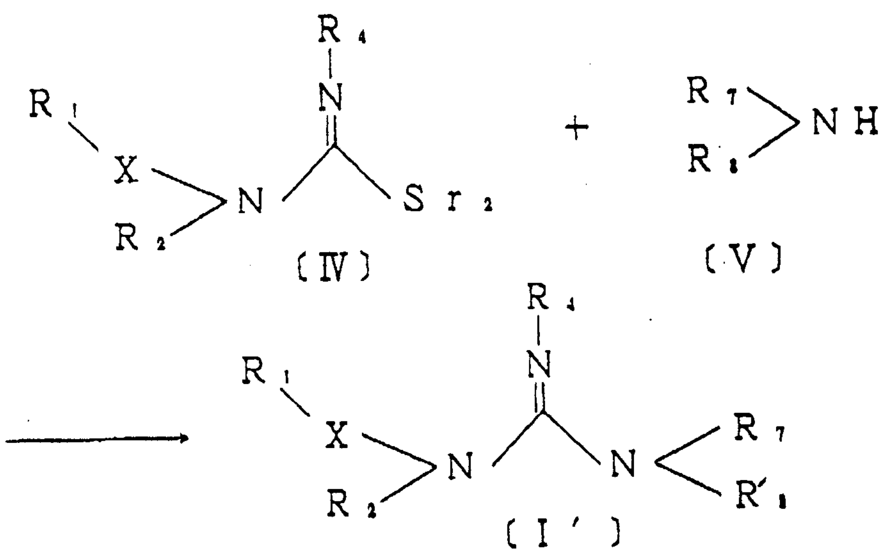

Wherein $r_2$ is lower alkyl; and X, $R_1$, $R_2$, $R_4$, $R_7$ and $R_8$ are same as above.

The reaction carried out under the same conditions as in the Process 1.

(3) Process 3:

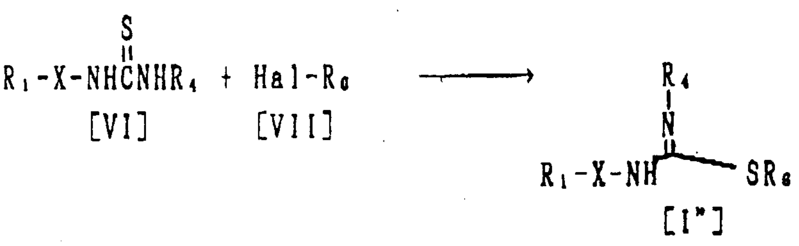

The reaction is carried out in inert solvent such as DMF, acetonitrile, acetone, benzene or xylene, if necessary under the presence for deacidifying agent. Potassium carbonate, sodium carb onate, triethylamine or DBU is applicable, at temperature range of room to boiling point of the solvent used.

(4) Process 4:In the case $R_3$ is $-SR_6$

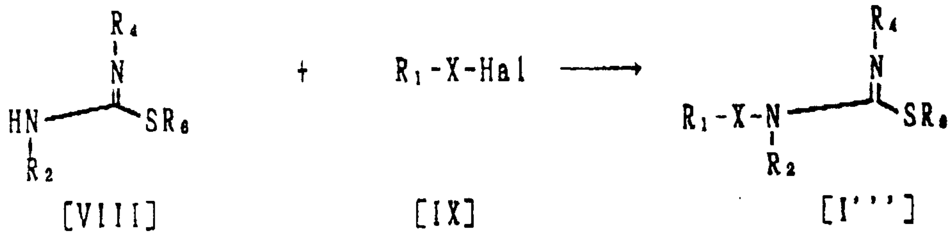

Wherein Hal is halogen; and R1, R2, R6 and X are same as above.

The reaction is desirably carried out in inert solvent, DMF under the presence for deacidifying agent. Potassium carbonate, sodium carbonate, NaH triethylamine or DBU is applicable, at room temperature,

(5) Process 5: In the case $R_3$ is $OR_6$

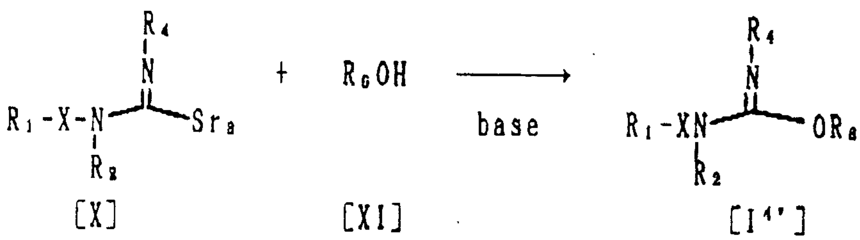


Wherein $r_3$ is lower alkyl and $R_1$, $R_2$, $R_4$, $R_6$, and X are same above.

The reaction is desirably carried out in inert solvent such as DMF or HF under the presence of base such as DBU, NaH or sodium methylate at room temperature or under refluxing condition. Other than above condition for using inert solvent, it is allowable to concurrently use the reagent $R_6OH$.

(6) Process 6: In the case $R_2$ is $-Y-R_5$ and Y is S(O)n, -CO- or $-CO_2-$

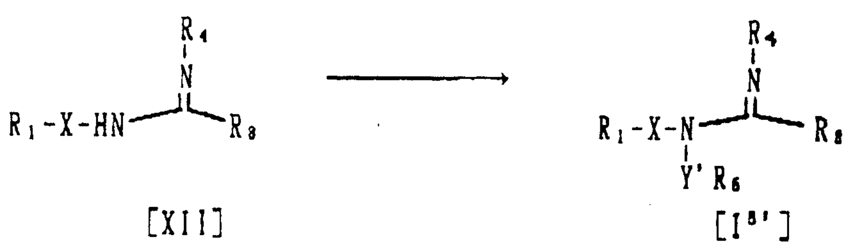

The desirable compound is obtainable by heating $R_5S(O)nCl$, $R_5COCl$, $ClCOOR_5$, $(R_5CO)_2O$ or the other analogues in aromatic hydrocarbon solvent, desirably such as benzene, toluene, xylene at -20 C to 200 C, desirably from room temperature to boiling point of the solvent. The reaction carried out, if necessary under the presence of deacidifying agent. Potassium carbonate, sodium carbonate, triethylamine, DBU or the other analogues are applicable.

(7) Process 7: In the case $R_2$ and $R_3$ form a ring by binding in each other.

(a)

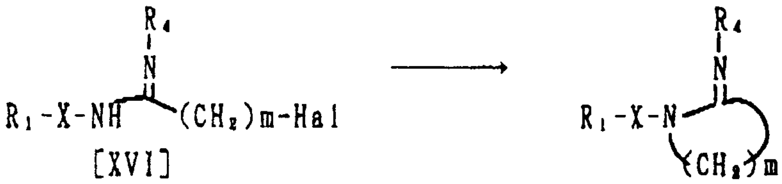

[XVI] [I6']

Wherein m is 2, 3 or 4; and Hal, X, $R_1$ and $R_4$ are same as above.

The reaction is similarly carried out as in process 3.

(b)

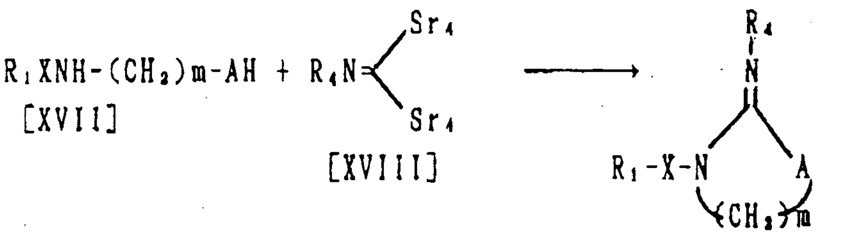

[XVII] [XVIII] [I7']

Wherein m is 2, 3 or 4; A is O, or S or NH; $r_4$ is lower alkyl; and X, $R_1$ and $R_4$ are same as above.

The reaction is similarly carried out in process 1.

Desirable substance can be obtained by the common treatment in all cases described after the reaction.

The compounds were identified by IR, NMR and MASS spectra and the other techniques. If $R_2$ in the invented compound is hydrogen the existence of tautomeric isomers represented by following equation is possible.

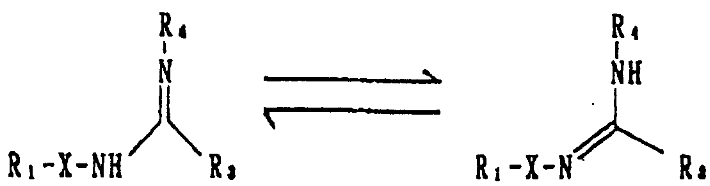

If $R_3$ is $NR_7$ $R_8$ and one of $R_7$ or $R_8$ is, at least, hydrogen the existence of tautomeric isomers represented by following equation is also possible.

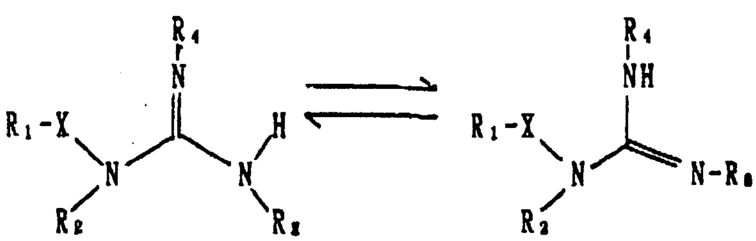

The existence of syn-anti isomers of the invented compounds represented following equation is possible, but the syn/anti ratio depends upon the instrumental analysis conditions.

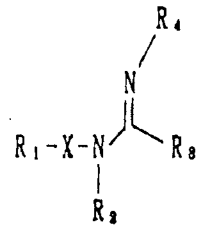

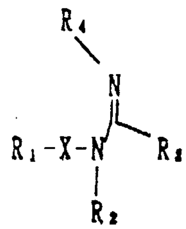

The Best Form to Execute the Invention

The invention is explained more detail with giving examples.

Reference Example 1

Methyl N-(2-thiazolyl) acetimidate;

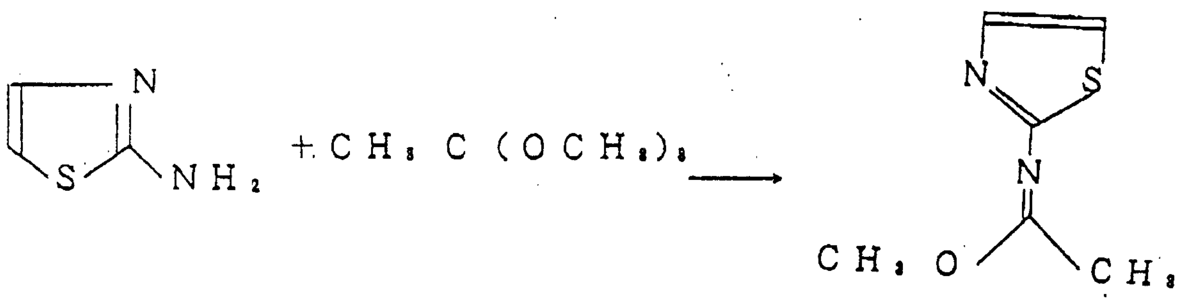

Ten grams of 2-aminothiazole and 13,4 g of methyl orthoacetate was mixed and heated at about 100 ° C with removing formed methanol out of the reaction system for 1 hour. After completing the reaction, 13 g of desired compound was obtained by distillation (95-96 ° C/15 mm Hg).

Example 1

N-(2-chloro-5-pyridylmethyl)-N'-2-thiazolylacetamidine (compound No. 2):

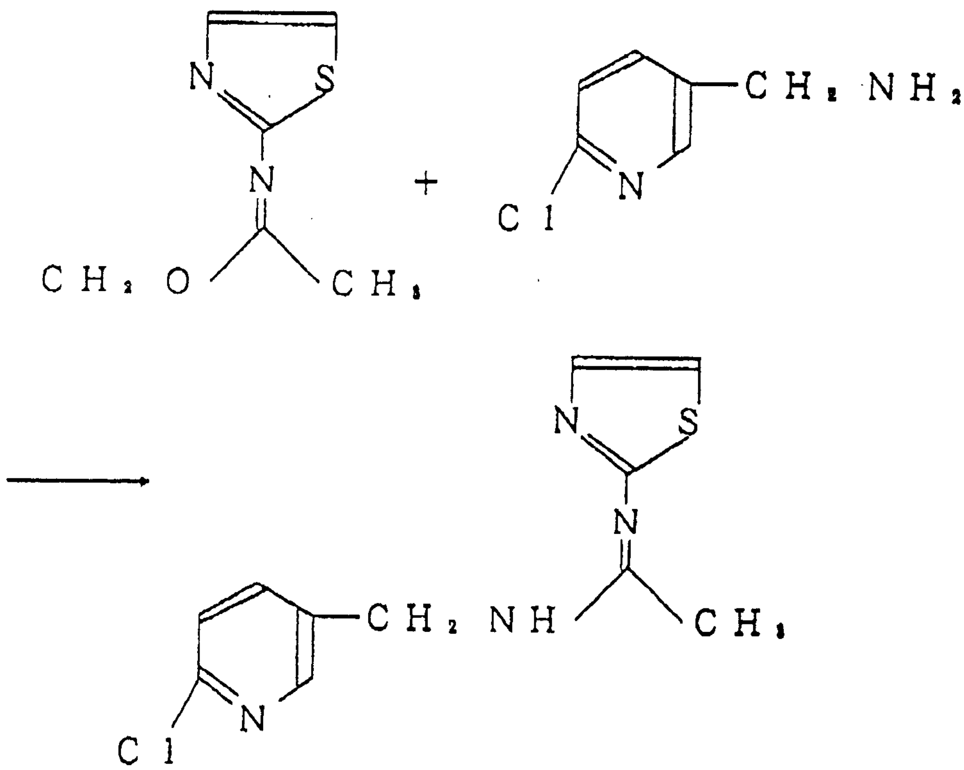

In 20 ml of ethanol 1.0 g of methyl N-(2-thiazolyl) acetimidate and 0.9 g of 2-chloro-5-pyridylmethylamine were added and refluxed for 2 hours. After the reaction 1.3 g of the desired compound was separated and refined from the reaction mixture through column chromatography, $n_D^{25}$ 1.6275.

Example 2

1-(2-Chloropyridylmethyl)-2-(thiazolyl-2-ylimino) imidazolidine (compound No. 455)

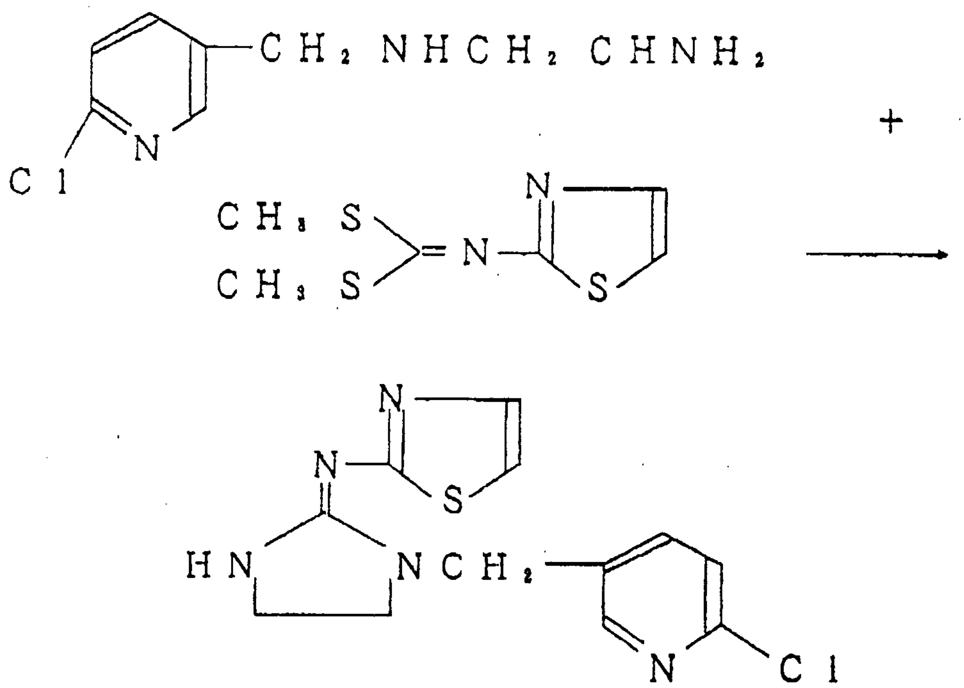

In 20 ml of ethanol 2.0 g of S,S'-dimethyl N-(2-thiazolyl) dithioiminocarbonate and 2,0 g of 2-chloro-5-pyridylmethylaminoethylamine were added and refluxed for 18 hours. After the reaction 1.6 g of the desired compound was separated and refined from the reaction mixture through column chromatography, m.p. 135 ° C.

Example 3

N-2-Chloro-5-pyridylmethyl-N'-methyl-N''-4-methyl-2-thiazolylguanidine (compound No. 226)

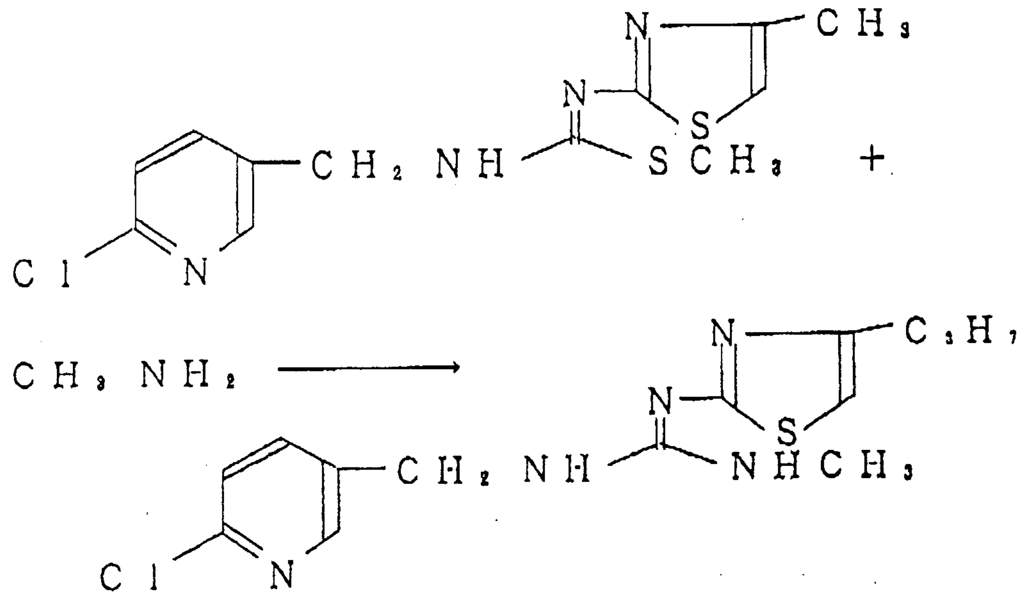

In 20 ml of ethanol 1.1 g of N-2-chloro-5-pyridylmethyl-N'-4methyl-2-thiazolyl-S-methyl isothiourea and o.6 g of 30 % methylamine (in ethanol solution) were added and refluxed overnight. After the reaction 0.8 g of the desired compound was separated and refined from the reaction mixture through column chromatography, m.p. 93-96 ° C.

The representative examples including above described compounds in the invention are shown in Table 1.

Table 1

Structure Formula

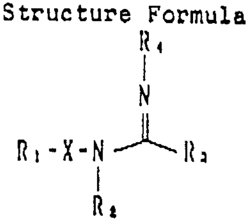

| Compound No. | $R_1$ X | $R_2$ | $R_3$ | $R_4$ | Physical Properties [] m.p.°C |
|---|---|---|---|---|---|
| 1 | 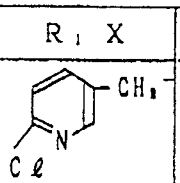 | H | H | 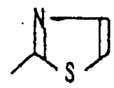 | [96-97] |
| 2 | 〃 | 〃 | $CH_3$ | 〃 | $n_D^{21}$ 1.6275 |
| 3 | 〃 | 〃 | $C_2H_5$ | 〃 | |
| 4 | 〃 | 〃 | $C_3H_7(n)$ | 〃 | |
| 5 | 〃 | 〃 | $CH<^{CH_3}_{CH_3}$ | 〃 | |
| 6 | 〃 | 〃 | 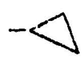 | 〃 | |
| 7 | 〃 | 〃 | $CCl_3$ | 〃 | |
| 8 | 〃 | 〃 | $CF_3$ | 〃 | |
| 9 | 〃 | 〃 | $CH_2Cl$ | 〃 | |
| 10 | 〃 | 〃 | $CH_2F$ | 〃 | |
| 11 | 〃 | 〃 | $CH_2CH_2Cl$ | 〃 | |
| 12 | 〃 | 〃 | $-CHCH_3$ (Cl on CH) | 〃 | |
| 13 | 〃 | 〃 | $CH_2CN$ | 〃 | |
| 14 | 〃 | 〃 | $CH_2CH_2CN$ | 〃 | |

| | | | | | |
|---|---|---|---|---|---|
| 15 | 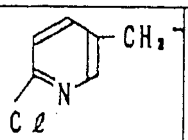 | H | $CH_2COOC_2H_5$ | 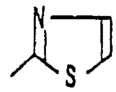 | |
| 16 | 〃 | 〃 | $CH_2OCH_3$ | 〃 | |
| 17 | 〃 | 〃 | $CH_2SCH_3$ | 〃 | |
| 18 | 〃 | 〃 | 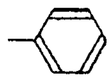 | 〃 | |
| 19 | 〃 | $CH_3$ | H | 〃 | $n_D^{21}$ 1.6478 |
| 20 | 〃 | 〃 | $CH_3$ | 〃 | [74-77] |
| 21 | 〃 | 〃 | $C_2H_5$ | 〃 | |
| 22 | 〃 | 〃 | $C_3H_7(n)$ | 〃 | |
| 23 | 〃 | 〃 | 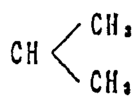 | 〃 | |
| 24 | 〃 | 〃 | 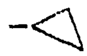 | 〃 | |
| 25 | 〃 | 〃 | $CCl_3$ | 〃 | |
| 26 | 〃 | 〃 | $CF_3$ | 〃 | |
| 27 | 〃 | 〃 | $CH_2Cl$ | 〃 | |
| 28 | 〃 | 〃 | $CH_2F$ | 〃 | |
| 29 | 〃 | 〃 | $CH_2CH_2Cl$ | 〃 | |
| 30 | 〃 | 〃 | 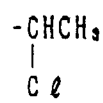 | 〃 | |
| 31 | 〃 | 〃 | $CH_2CN$ | 〃 | |
| 32 | 〃 | 〃 | $CH_2CH_2CN$ | 〃 | |
| 33 | 〃 | 〃 | $CH_2COOC_2H_5$ | 〃 | |

| | | | | | |
|---|---|---|---|---|---|
| 34 | 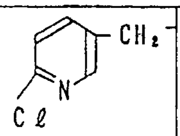 | $CH_3$ | $CH_2OCH_3$ | 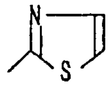 | |
| 35 | 〃 | 〃 | $CH_2SCH_3$ | 〃 | |
| 36 | 〃 | 〃 | 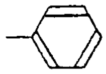 | 〃 | |
| 37 | 〃 | 〃 | $-CH=CH_2$ | 〃 | |
| 38 | 〃 | $C_2H_5$ | H | 〃 | |
| 39 | 〃 | 〃 | $CH_3$ | 〃 | |
| 40 | 〃 | $C_3H_7(n)$ | H | 〃 | |
| 41 | 〃 | 〃 | $CH_3$ | 〃 | |
| 42 | 〃 | $C_3H_7(i)$ | H | 〃 | |
| 43 | 〃 | 〃 | $CH_3$ | 〃 | |
| 44 | 〃 | 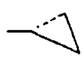 | H | 〃 | |
| 45 | 〃 | 〃 | $CH_3$ | 〃 | |
| 46 | 〃 | $CH_2CH=CH_2$ | H | 〃 | |
| 47 | 〃 | 〃 | $CH_3$ | 〃 | |
| 48 | 〃 | $CH_2C\equiv CH$ | H | 〃 | |
| 49 | 〃 | 〃 | $CH_3$ | 〃 | |
| 50 | 〃 | 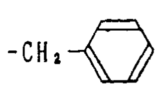 | H | 〃 | |
| 51 | 〃 | 〃 | $CH_3$ | 〃 | |

| | | | | | |
|---|---|---|---|---|---|
| 52 | 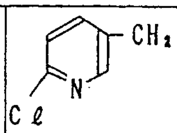 | CHO | H | 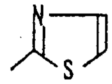 | |
| 53 | 〃 | 〃 | $CH_3$ | 〃 | |
| 54 | 〃 | $COCH_3$ | H | 〃 | |
| 55 | 〃 | 〃 | $CH_3$ | 〃 | |
| 56 | 〃 | $SO_2CH_3$ | H | 〃 | |
| 57 | 〃 | 〃 | $CH_3$ | 〃 | |
| 58 | 〃 | H | $CH_3$ | 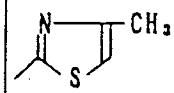 | $n_D^{24.5}$ 1.6220 |
| 59 | 〃 | $CH_3$ | 〃 | 〃 | $n_D^{24.5}$ 1.6139 |
| 60 | 〃 | H | 〃 | 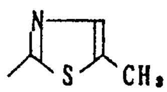 | [94-96] |
| 61 | 〃 | $CH_3$ | 〃 | 〃 | [65-66] |
| 62 | 〃 | H | 〃 | 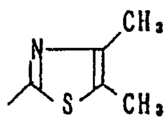 | $n_D^{27}$ 1.6240 |
| 63 | 〃 | $CH_3$ | 〃 | 〃 | $n_D^{27}$ 1.6061 |
| 64 | 〃 | H | 〃 | 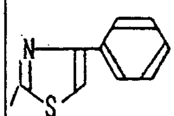 | [95-96] |
| 65 | 〃 | $CH_3$ | 〃 | 〃 | [94-96] |
| 66 | 〃 | H | 〃 | 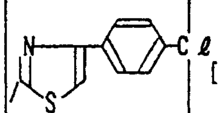 | [143-145] |
| 67 | 〃 | $CH_3$ | 〃 | 〃 | [89-91] |
| 68 | 〃 | H | 〃 | 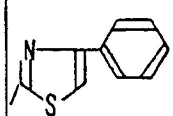 | [102-104] |
| 69 | 〃 | $CH_3$ | 〃 | 〃 | [95-97] |

| | | | | | |
|---|---|---|---|---|---|
| 70 | 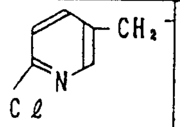 | H | $CH_3$ | 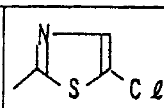 | |
| 71 | 〃 | $CH_3$ | 〃 | 〃 | |
| 72 | 〃 | H | 〃 | 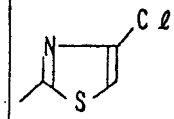 | |
| 73 | 〃 | $CH_3$ | 〃 | 〃 | |
| 74 | 〃 | H | 〃 | 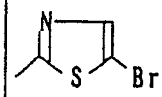 | |
| 75 | 〃 | $CH_3$ | 〃 | 〃 | |
| 76 | 〃 | H | 〃 | 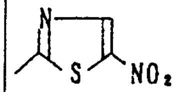 | |
| 77 | 〃 | $CH_3$ | 〃 | 〃 | |
| 78 | 〃 | H | 〃 | 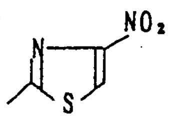 | |
| 79 | 〃 | $CH_3$ | 〃 | 〃 | |
| 80 | 〃 | H | 〃 | 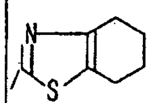 | |
| 81 | 〃 | $CH_3$ | 〃 | 〃 | |
| 82 | 〃 | H | 〃 | 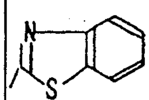 | $n_D^{24.6}$ 1.6660 |
| 83 | 〃 | $CH_3$ | 〃 | 〃 | [90-91.5] |
| 84 | 〃 | H | 〃 | 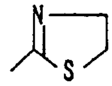 | |
| 85 | 〃 | $CH_3$ | 〃 | 〃 | |
| 86 | 〃 | H | 〃 | 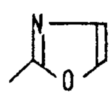 | |

| | | | | | |
|---|---|---|---|---|---|
| 87 | 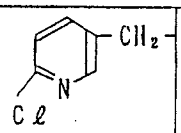 | $CH_3$ | $CH_3$ | 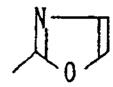 | |
| 88 | 〃 | H | 〃 | 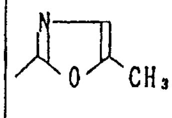 | |
| 89 | 〃 | $CH_3$ | 〃 | 〃 | |
| 90 | 〃 | H | 〃 | 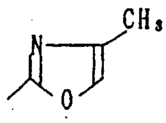 | $n_D^{25}$ 1.5861 |
| 91 | 〃 | $CH_3$ | 〃 | 〃 | $n_D^{25}$ 1.5880 |
| 92 | 〃 | H | 〃 | 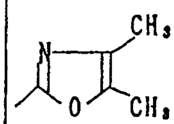 | |
| 93 | 〃 | $CH_3$ | 〃 | 〃 | |
| 94 | 〃 | H | 〃 | 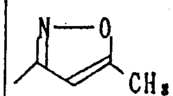 | [122-124] |
| 95 | 〃 | $CH_3$ | 〃 | 〃 | |
| 96 | 〃 | H | 〃 | 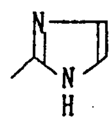 | |
| 97 | 〃 | $CH_3$ | 〃 | 〃 | |
| 98 | 〃 | H | 〃 | 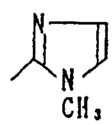 | |
| 99 | 〃 | $CH_3$ | 〃 | 〃 | |
| 100 | 〃 | H | 〃 | 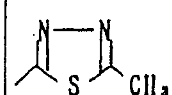 | [109-110] |
| 101 | 〃 | $CH_3$ | 〃 | 〃 | $n_D^{26.5}$ 1.6142 |
| 102 | 〃 | H | 〃 | 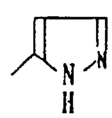 | |
| 103 | 〃 | 〃 | 〃 | 〃 | |

| | | | | | |
|---|---|---|---|---|---|
| 104 | 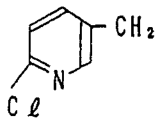 | H | $CH_3$ | 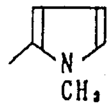 | |
| 105 | 〃 | $CH_3$ | 〃 | 〃 | |
| 106 | 〃 | H | 〃 | 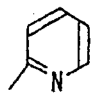 | $n_D^{25}$ 1.5942 |
| 107 | 〃 | $CH_3$ | 〃 | 〃 | $n_D^{24.5}$ 1.6065 |
| 108 | 〃 | H | 〃 | 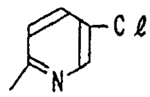 | $n_D^{26.5}$ 1.6075 |
| 109 | 〃 | $CH_3$ | 〃 | 〃 | [88-90] |
| 110 | 〃 | H | 〃 | 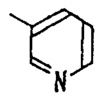 | $n_D^{24.5}$ 1.5881 |
| 111 | 〃 | $CH_3$ | 〃 | 〃 | |
| 112 | 〃 | H | 〃 | 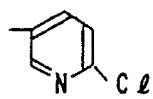 | |
| 113 | 〃 | $CH_3$ | 〃 | 〃 | |
| 114 | 〃 | H | 〃 | 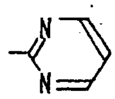 | |
| 115 | 〃 | $CH_3$ | 〃 | 〃 | $n_D^{25.5}$ 1.6053 |
| 116 | 〃 | H | 〃 | 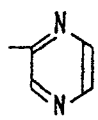 | |
| 117 | 〃 | $CH_3$ | 〃 | 〃 | |
| 118 | 〃 | H | 〃 | 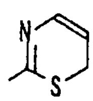 | |
| 119 | 〃 | $CH_3$ | 〃 | 〃 | |
| 120 | 〃 | H | 〃 | 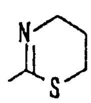 | |
| 121 | 〃 | $CH_3$ | 〃 | 〃 | |

| | | | | | |
|---|---|---|---|---|---|
| 122 | 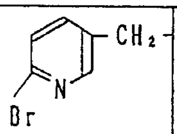 | H | $CH_3$ | 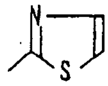 | |
| 123 | 〃 | $CH_3$ | 〃 | 〃 | |
| 124 | 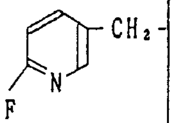 | H | 〃 | 〃 | |
| 125 | 〃 | $CH_3$ | 〃 | 〃 | |
| 126 | 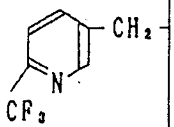 | H | 〃 | 〃 | |
| 127 | 〃 | $CH_3$ | 〃 | 〃 | |
| 128 | 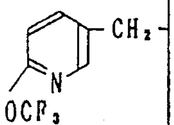 | H | 〃 | 〃 | |
| 129 | 〃 | $CH_3$ | 〃 | 〃 | |
| 130 | 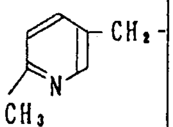 | H | 〃 | 〃 | |
| 131 | 〃 | $CH_3$ | 〃 | 〃 | |
| 132 | 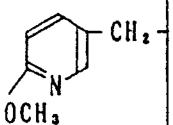 | H | 〃 | 〃 | |
| 133 | 〃 | $CH_3$ | 〃 | 〃 | |
| 134 | 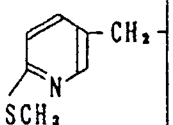 | H | 〃 | 〃 | |
| 135 | 〃 | $CH_3$ | 〃 | 〃 | |
| 136 | 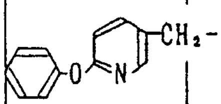 | H | 〃 | 〃 | |
| 137 | 〃 | $CH_3$ | 〃 | 〃 | |
| 138 | 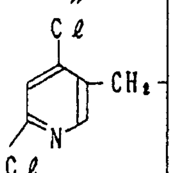 | H | 〃 | 〃 | |
| 139 | 〃 | $CH_3$ | 〃 | 〃 | |

| | | | | | |
|---|---|---|---|---|---|
| 140 | 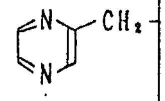 | H | $CH_3$ | 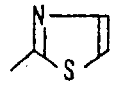 | |
| 141 | 〃 | $CH_3$ | 〃 | 〃 | [104-105] |
| 142 | 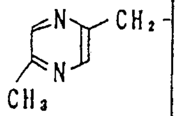 | H | 〃 | 〃 | |
| 143 | 〃 | $CH_3$ | 〃 | 〃 | $n_D^{24.5}$ 1.6125 |
| 144 | 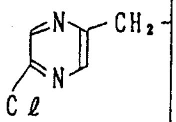 | H | 〃 | 〃 | |
| 145 | 〃 | $CH_3$ | 〃 | 〃 | |
| 146 | 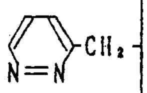 | H | 〃 | 〃 | |
| 147 | 〃 | $CH_3$ | 〃 | 〃 | |
| 148 | 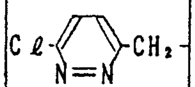 | H | 〃 | 〃 | |
| 149 | 〃 | $CH_3$ | 〃 | 〃 | |
| 150 | 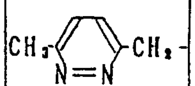 | H | 〃 | 〃 | |
| 151 | 〃 | $CH_3$ | 〃 | 〃 | |
| 152 | 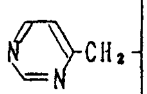 | H | 〃 | 〃 | |
| 153 | 〃 | $CH_3$ | 〃 | 〃 | |
| 154 | 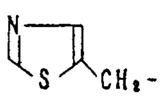 | H | 〃 | 〃 | |
| 155 | 〃 | $CH_3$ | 〃 | 〃 | |
| 156 | 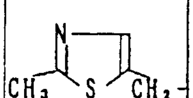 | H | 〃 | 〃 | |
| 157 | 〃 | $CH_3$ | 〃 | 〃 | |

| | | | | | |
|---|---|---|---|---|---|
| 158 | 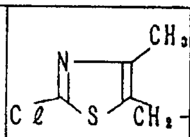 | H | $CH_3$ | 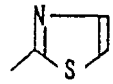 | $n_D^{25}$ 1.6130 |
| 159 | 〃 | $CH_3$ | 〃 | 〃 | |
| 160 | 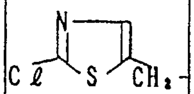 | H | 〃 | 〃 | [97-98] |
| 161 | 〃 | $CH_3$ | 〃 | 〃 | [98-100] |
| 162 | 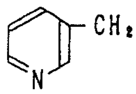 | H | 〃 | 〃 | |
| 163 | 〃 | $CH_3$ | 〃 | 〃 | |
| 164 | 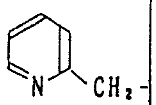 | H | 〃 | 〃 | |
| 165 | 〃 | $CH_3$ | 〃 | 〃 | |
| 166 | 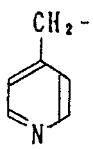 | H | 〃 | 〃 | |
| 167 | 〃 | $CH_3$ | 〃 | 〃 | |
| 168 | 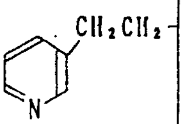 | H | 〃 | 〃 | |
| 169 | 〃 | $CH_3$ | 〃 | 〃 | |
| 170 | 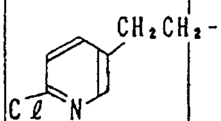 | H | 〃 | 〃 | |
| 171 | 〃 | $CH_3$ | 〃 | 〃 | |

| | | | | | |
|---|---|---|---|---|---|
| 172 | 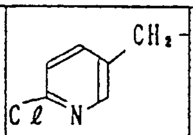 | H | $NH_2$ | 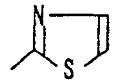 | |
| 173 | 〃 | 〃 | $NHCOCH_3$ | 〃 | |
| 174 | 〃 | 〃 | $NHSO_2CH_3$ | 〃 | |
| 175 | 〃 | 〃 | $NHCH_3$ | 〃 | [66-69] |
| 176 | 〃 | 〃 | $NHC_2H_5$ | 〃 | |
| 177 | 〃 | 〃 | $NHCH\langle\begin{smallmatrix}CH_3\\CH_3\end{smallmatrix}$ | 〃 | |
| 178 | 〃 | 〃 | $NHCH_2CH=CH_2$ | 〃 | |
| 179 | 〃 | 〃 | $NHCH_2C\equiv CH$ | 〃 | |
| 180 | 〃 | 〃 | $NHCH_2CH_2Cl$ | 〃 | |
| 181 | 〃 | 〃 | $NHCH_2CH_2CN$ | 〃 | |
| 182 | 〃 | 〃 | $NHCH_2COOC_2H_5$ | 〃 | |
| 183 | 〃 | 〃 | HN 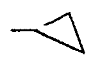 | 〃 | |
| 184 | 〃 | 〃 | $NHCH_2$- 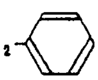 | 〃 | |
| 185 | 〃 | 〃 | $N\langle\begin{smallmatrix}CH_3\\CH_3\end{smallmatrix}$ | 〃 | |
| 186 | 〃 | 〃 | 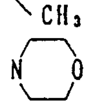 | 〃 | |
| 187 | 〃 | 〃 | $N\langle\begin{smallmatrix}CH_3\\COCH_3\end{smallmatrix}$ | 〃 | |
| 188 | 〃 | 〃 | $N\langle\begin{smallmatrix}CH_3\\SO_2CH_3\end{smallmatrix}$ | 〃 | |

| | | | | | |
|---|---|---|---|---|---|
| 189 | 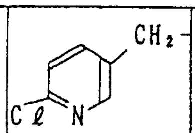 | $CH_3$ | $NH_2$ | 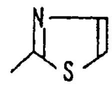 | |
| 190 | 〃 | 〃 | $NHCOCH_3$ | 〃 | |
| 191 | 〃 | 〃 | $NHSO_2CH_3$ | 〃 | |
| 192 | 〃 | 〃 | $NHCH_3$ | 〃 | |
| 193 | 〃 | 〃 | $NHC_2H_5$ | 〃 | |
| 194 | 〃 | 〃 | 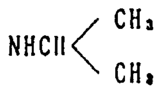 | 〃 | |
| 195 | 〃 | 〃 | $NHCH_2CH{=}CH_2$ | 〃 | |
| 196 | 〃 | 〃 | $NHCH_2C{\equiv}CH$ | 〃 | |
| 197 | 〃 | 〃 | $NHCH_2CH_2Cl$ | 〃 | |
| 198 | 〃 | 〃 | $NHCH_2CH_2CN$ | 〃 | |
| 199 | 〃 | 〃 | $NHCH_2COOC_2H_5$ | 〃 | |
| 200 | 〃 | 〃 | NH 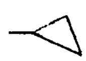 | 〃 | |
| 201 | 〃 | 〃 | $NHCH_2$- 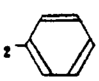 | 〃 | |
| 202 | 〃 | 〃 | 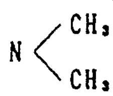 | 〃 | |
| 203 | 〃 | 〃 | 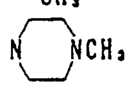 | 〃 | |
| 204 | 〃 | 〃 | 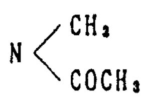 | 〃 | |
| 205 | 〃 | 〃 | 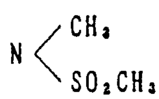 | 〃 | |

| | | | | | |
|---|---|---|---|---|---|
| 206 | 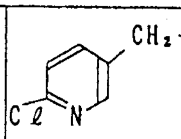 | $C_2H_5$ | $NHCH_3$ | 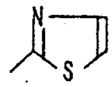 | |
| 207 | 〃 | 〃 | $NHC_2H_5$ | 〃 | |
| 208 | 〃 | 〃 | $N\langle^{CH_3}_{CH_3}$ | 〃 | |
| 209 | 〃 | $CH\langle^{CH_3}_{CH_3}$ | $NHCH_3$ | 〃 | |
| 210 | 〃 | 〃 | $NHC_2H_5$ | 〃 | |
| 211 | 〃 | 〃 | $N\langle^{CH_3}_{CH_3}$ | 〃 | |
| 212 | 〃 | 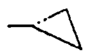 | $NHCH_3$ | 〃 | |
| 213 | 〃 | 〃 | $NHC_2H_5$ | 〃 | |
| 214 | 〃 | 〃 | $N\langle^{CH_3}_{CH_3}$ | 〃 | |
| 215 | 〃 | $CH_2CH{=}CH_2$ | $NHCH_3$ | 〃 | |
| 216 | 〃 | 〃 | $NHC_2H_5$ | 〃 | |
| 217 | 〃 | 〃 | $N\langle^{CH_3}_{CH_3}$ | 〃 | |
| 218 | 〃 | $CH_2C\equiv CH$ | $NHCH_3$ | 〃 | |
| 219 | 〃 | 〃 | $NHC_2H_5$ | 〃 | |
| 220 | 〃 | 〃 | $-N\langle^{CH_3}_{CH_3}$ | 〃 | |
| 221 | 〃 | $COCH_3$ | $NHCH_3$ | 〃 | |
| 222 | 〃 | 〃 | $NHC_2H_5$ | 〃 | |
| 223 | 〃 | 〃 | $N\langle^{CH_3}_{CH_3}$ | 〃 | |

| | | | | | |
|---|---|---|---|---|---|
| 224 | 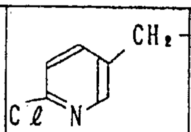 | $SO_2CH_3$ | $NHCH_3$ | 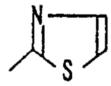 | |
| 225 | 〃 | 〃 | $NHC_2H_5$ | 〃 | |
| 226 | 〃 | H | $NHCH_3$ | 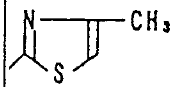 | [93-96] |
| 227 | 〃 | 〃 | $NHC_2H_5$ | 〃 | $n_D^{25}$1.5958 |
| 228 | 〃 | 〃 | $NHCH_2CH=CH_2$ | 〃 | $n_D^{24.5}$1.6094 |
| 229 | 〃 | 〃 | 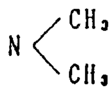 | 〃 | $n_D^{25}$1.6065 |
| 230 | 〃 | 〃 | $NHCH_3$ | 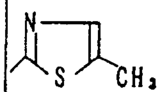 | [118-119] |
| 231 | 〃 | 〃 | 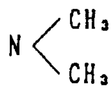 | 〃 | |
| 232 | 〃 | 〃 | $NHCH_3$ | 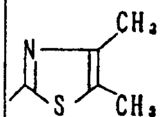 | [132-133.5] |
| 233 | 〃 | 〃 | 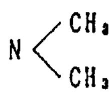 | 〃 | |
| 234 | 〃 | 〃 | $NHCH_3$ | 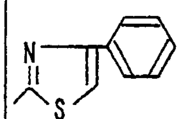 | [129-132] |
| 235 | 〃 | 〃 | 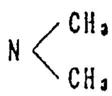 | 〃 | |
| 236 | 〃 | 〃 | $NHCH_3$ | 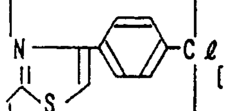 | [168-170] |
| 237 | 〃 | 〃 | 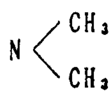 | 〃 | |
| 238 | 〃 | 〃 | $NHCH_3$ | 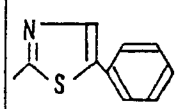 | [139-140] |
| 239 | 〃 | 〃 | 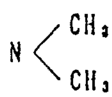 | 〃 | |

| | | | | | |
|---|---|---|---|---|---|
| 240 | 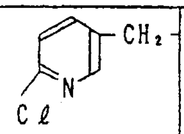 | H | $NHCH_3$ | 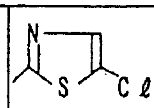 | $n_D^{25}$ 1.6215 |
| 241 | 〃 | 〃 | 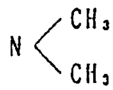 | 〃 | |
| 242 | 〃 | 〃 | $NHCH_3$ | 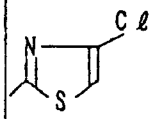 | |
| 243 | 〃 | 〃 | 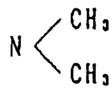 | 〃 | |
| 244 | 〃 | 〃 | $NHCH_3$ | 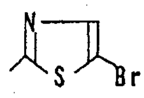 | |
| 245 | 〃 | 〃 | 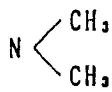 | 〃 | |
| 246 | 〃 | 〃 | $NHCH_3$ | 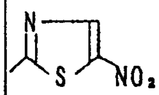 | [221-224] |
| 247 | 〃 | 〃 | 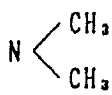 | 〃 | |
| 248 | 〃 | 〃 | $NHCH_3$ | 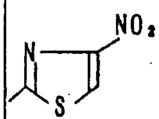 | |
| 249 | 〃 | 〃 | 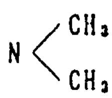 | 〃 | |
| 250 | 〃 | 〃 | $NHCH_3$ | 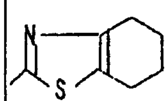 | |
| 251 | 〃 | 〃 | 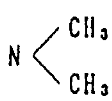 | 〃 | |
| 252 | 〃 | 〃 | $NHCH_3$ | 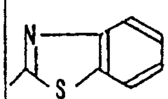 | |
| 253 | 〃 | 〃 | 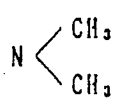 | 〃 | |
| 254 | 〃 | 〃 | $NHCH_3$ | 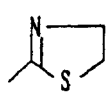 | |
| 255 | 〃 | 〃 | 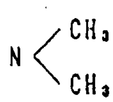 | 〃 | |

| | | | | | |
|---|---|---|---|---|---|
| 256 | 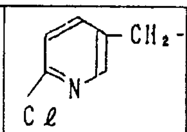 | H | NHCH₃ | 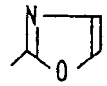 | |
| 257 | 〃 | 〃 | 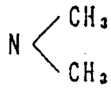 | 〃 | |
| 258 | 〃 | 〃 | NHCH₃ | 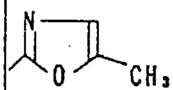 | |
| 259 | 〃 | 〃 | 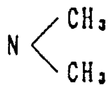 | 〃 | |
| 260 | 〃 | 〃 | NHCH₃ | 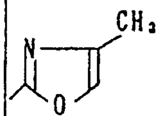 | |
| 261 | 〃 | 〃 | 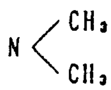 | 〃 | |
| 262 | 〃 | 〃 | NHCH₃ | 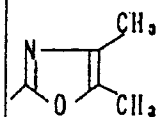 | |
| 263 | 〃 | 〃 | 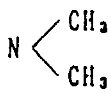 | 〃 | |
| 264 | 〃 | 〃 | NHCH₃ | 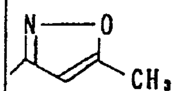 | |
| 265 | 〃 | 〃 | 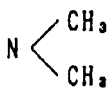 | 〃 | |
| 266 | 〃 | 〃 | NHCH₃ | 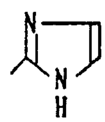 | [148-150] |
| 267 | 〃 | 〃 | 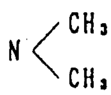 | 〃 | |
| 268 | 〃 | 〃 | NHCH₃ | 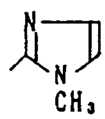 | |
| 269 | 〃 | 〃 | 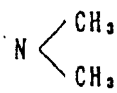 | 〃 | |
| 270 | 〃 | 〃 | NHCH₃ | 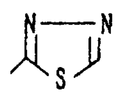 | |
| 271 | 〃 | 〃 | 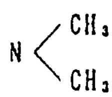 | 〃 | |

| | | | | | |
|---|---|---|---|---|---|
| 272 | 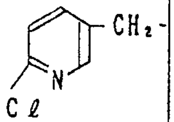 | H | $NHCH_3$ | 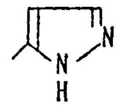 | |
| 273 | 〃 | 〃 | $N(CH_3)_2$ | 〃 | |
| 274 | 〃 | 〃 | $NHCH_3$ | 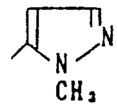 | |
| 275 | 〃 | 〃 | $N(CH_3)_2$ | 〃 | |
| 276 | 〃 | 〃 | $NHCH_3$ | 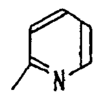 | $n_D^{23}$ 1.6428 |
| 277 | 〃 | 〃 | $N(CH_3)_2$ | 〃 | |
| 278 | 〃 | 〃 | $NHCH_3$ | 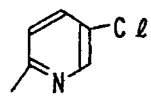 | |
| 279 | 〃 | 〃 | $N(CH_3)_2$ | 〃 | |
| 280 | 〃 | 〃 | $NHCH_3$ | 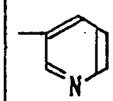 | |
| 281 | 〃 | 〃 | $N(CH_3)_2$ | 〃 | |
| 282 | 〃 | 〃 | $NHCH_3$ | 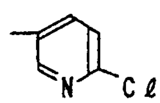 | [143-145] |
| 283 | 〃 | 〃 | $N(CH_3)_2$ | 〃 | |
| 284 | 〃 | 〃 | $NHCH_3$ | 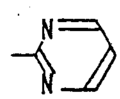 | $n_D^{25}$ 1.6294 |
| 285 | 〃 | 〃 | $N(CH_3)_2$ | 〃 | |
| 286 | 〃 | 〃 | $NHCH_3$ | 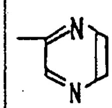 | |
| 287 | 〃 | 〃 | $N(CH_3)_2$ | 〃 | |
| 288 | 〃 | 〃 | $NHCH_3$ | 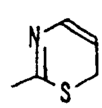 | |

| | | | | | |
|---|---|---|---|---|---|
| 289 | 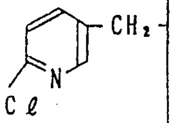 | H | 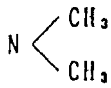 | 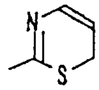 | |
| 290 | 〃 | 〃 | $NHCH_3$ | 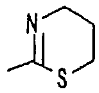 | |
| 291 | 〃 | 〃 | 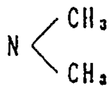 | 〃 | |
| 292 | 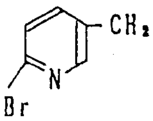 | 〃 | $NHCH_3$ | 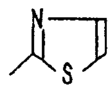 | |
| 293 | 〃 | $CH_3$ | 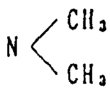 | 〃 | |
| 294 | 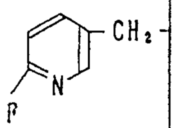 | H | $NHCH_3$ | 〃 | |
| 295 | 〃 | $CH_3$ | 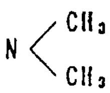 | 〃 | |
| 296 | 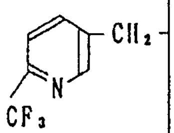 | H | $NHCH_3$ | 〃 | |
| 297 | 〃 | $CH_3$ | 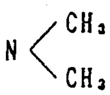 | 〃 | |
| 298 | 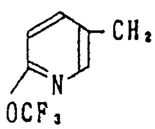 | H | $NHCH_3$ | 〃 | |
| 299 | 〃 | $CH_3$ | 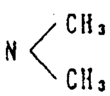 | 〃 | |
| 300 | 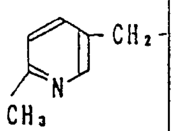 | H | $NHCH_3$ | 〃 | |
| 301 | 〃 | $CH_3$ | 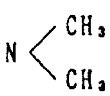 | 〃 | |
| 302 | 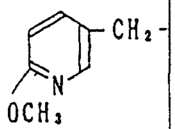 | H | $NHCH_3$ | 〃 | |

| | | | | | |
|---|---|---|---|---|---|
| 303 | 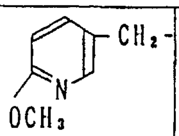 | $CH_3$ | 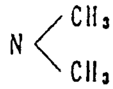 | 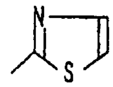 | |
| 304 | 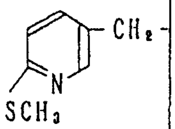 | H | $NHCH_3$ | 〃 | |
| 305 | 〃 | $CH_3$ | 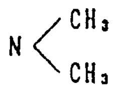 | 〃 | |
| 306 | 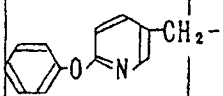 | H | $NHCH_3$ | 〃 | |
| 307 | 〃 | $CH_3$ | 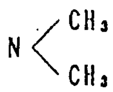 | 〃 | |
| 308 | 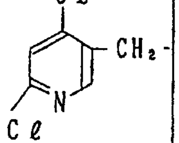 | H | $NHCH_3$ | 〃 | |
| 309 | 〃 | $CH_3$ | 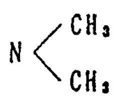 | 〃 | |
| 310 | 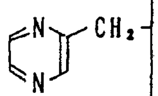 | H | $NHCH_3$ | 〃 | |
| 311 | 〃 | $CH_3$ | 〃 | 〃 | |
| 312 | 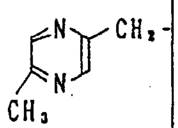 | H | 〃 | 〃 | |
| 313 | 〃 | $CH_3$ | 〃 | 〃 | |
| 314 | 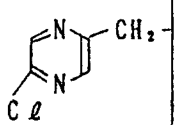 | H | 〃 | 〃 | |
| 315 | 〃 | $CH_3$ | 〃 | 〃 | |
| 316 | 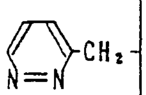 | H | 〃 | 〃 | |
| 317 | 〃 | $CH_3$ | 〃 | 〃 | |
| 318 | 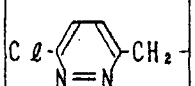 | H | 〃 | 〃 | |

| | | | | | |
|---|---|---|---|---|---|
| 319 | 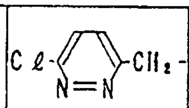 | $CH_3$ | $NHCH_3$ | 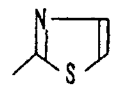 | |
| 320 | 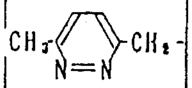 | H | 〃 | 〃 | |
| 321 | 〃 | $CH_3$ | 〃 | 〃 | |
| 322 | 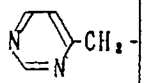 | H | 〃 | 〃 | |
| 323 | 〃 | $CH_3$ | 〃 | 〃 | |
| 324 | 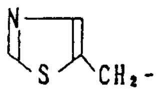 | H | 〃 | 〃 | |
| 325 | 〃 | $CH_3$ | 〃 | 〃 | |
| 326 | 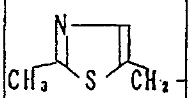 | H | 〃 | 〃 | |
| 327 | 〃 | $CH_3$ | 〃 | 〃 | |
| 328 | 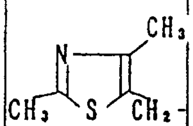 | H | 〃 | 〃 | |
| 329 | 〃 | $CH_3$ | 〃 | 〃 | |
| 330 | 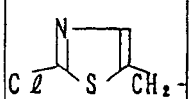 | H | 〃 | 〃 | |
| 331 | 〃 | $CH_3$ | 〃 | 〃 | |
| 332 | 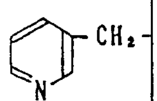 | H | 〃 | 〃 | |
| 333 | 〃 | $CH_3$ | 〃 | 〃 | |
| 334 | 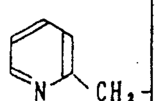 | H | 〃 | 〃 | |
| 335 | 〃 | $CH_3$ | 〃 | 〃 | |

| | | | | | |
|---|---|---|---|---|---|
| 336 | 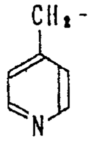 | H | $NHCH_3$ | 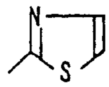 | |
| 337 | 〃 | $CH_3$ | 〃 | 〃 | |
| 338 | 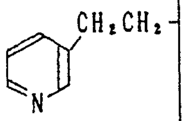 | H | 〃 | 〃 | |
| 339 | 〃 | $CH_3$ | 〃 | 〃 | |
| 340 | 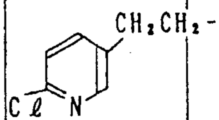 | H | 〃 | 〃 | |
| 341 | 〃 | $CH_3$ | 〃 | 〃 | |
| 342 | 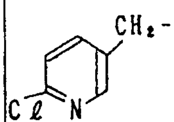 | H | $OCH_3$ | 〃 | |
| 343 | 〃 | 〃 | $OC_2H_5$ | 〃 | |
| 344 | 〃 | 〃 | $OCH_2CH=CH_2$ | 〃 | |
| 345 | 〃 | 〃 | $O-CH<^{CH_3}_{CH_3}$ | 〃 | |
| 346 | 〃 | 〃 | $OCH_2C\equiv CH$ | 〃 | |
| 347 | 〃 | 〃 | $OCH_2-$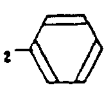 | 〃 | |
| 348 | 〃 | $CH_3$ | $OCH_3$ | 〃 | |
| 349 | 〃 | 〃 | $OC_2H_5$ | 〃 | |
| 350 | 〃 | $CH<^{CH_3}_{CH_3}$ | $OCH_3$ | 〃 | |
| 351 | 〃 | 〃 | $OC_2H_5$ | 〃 | |
| 352 | 〃 | 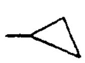 | $OCH_3$ | 〃 | |

| | | | | | |
|---|---|---|---|---|---|
| 353 | 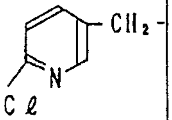 | 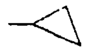 | $OC_2H_5$ | 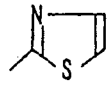 | |
| 354 | 〃 | $CH_2CH=CH_2$ | $OCH_3$ | 〃 | |
| 355 | 〃 | 〃 | $OC_2H_5$ | 〃 | |
| 356 | 〃 | $CH_2C\equiv CH$ | $OCH_3$ | 〃 | |
| 357 | 〃 | 〃 | $OC_2H_5$ | 〃 | |
| 358 | 〃 | $COCH_3$ | $OCH_3$ | 〃 | |
| 359 | 〃 | $SO_2CH_3$ | 〃 | 〃 | |
| 360 | 〃 | H | 〃 | 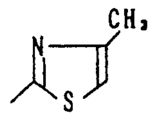 | |
| 361 | 〃 | 〃 | 〃 | 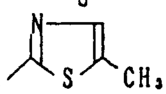 | |
| 362 | 〃 | 〃 | 〃 | 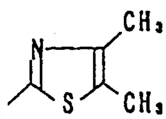 | |
| 363 | 〃 | 〃 | 〃 | 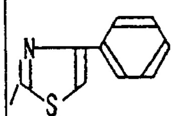 | |
| 364 | 〃 | 〃 | 〃 | 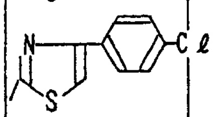 | |
| 365 | 〃 | 〃 | 〃 | 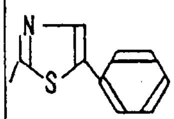 | |
| 366 | 〃 | 〃 | 〃 | 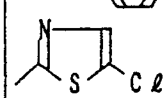 | |
| 367 | 〃 | 〃 | 〃 | 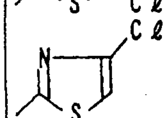 | |

| | | | | | |
|---|---|---|---|---|---|
| 368 | 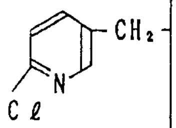 | H | $OCH_3$ | 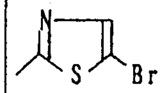 | |
| 369 | 〃 | 〃 | 〃 | 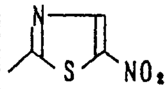 | |
| 370 | 〃 | 〃 | 〃 | 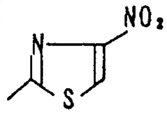 | |
| 371 | 〃 | 〃 | 〃 | 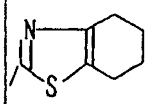 | |
| 372 | 〃 | 〃 | 〃 | 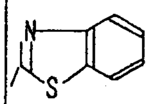 | |
| 373 | 〃 | 〃 | 〃 | 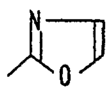 | |
| 374 | 〃 | 〃 | 〃 | 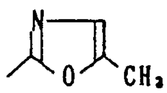 | |
| 375 | 〃 | 〃 | 〃 | 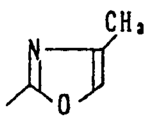 | |
| 376 | 〃 | 〃 | 〃 | 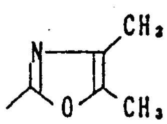 | |
| 377 | 〃 | 〃 | 〃 | 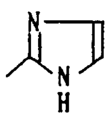 | |
| 378 | 〃 | 〃 | 〃 | 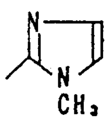 | |
| 379 | 〃 | 〃 | 〃 | 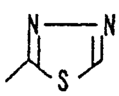 | |

| | | | | | |
|---|---|---|---|---|---|
| 380 | 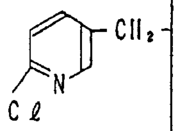 | H | $OCH_3$ | 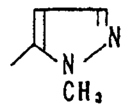 | |
| 381 | 〃 | 〃 | 〃 | 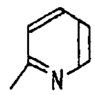 | |
| 382 | 〃 | 〃 | 〃 | 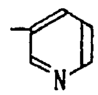 | |
| 383 | 〃 | 〃 | 〃 | 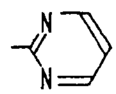 | |
| 384 | 〃 | 〃 | 〃 | 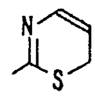 | |
| 385 | 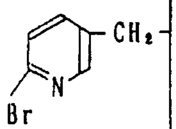 | 〃 | 〃 | 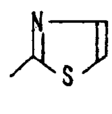 | |
| 386 | 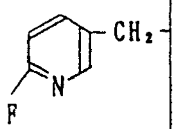 | 〃 | 〃 | 〃 | |
| 387 | 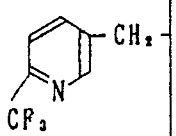 | 〃 | 〃 | 〃 | |
| 388 | 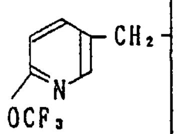 | 〃 | 〃 | 〃 | |
| 389 | 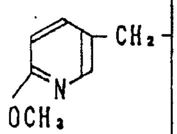 | 〃 | 〃 | 〃 | |

| | | | | | |
|---|---|---|---|---|---|
| 390 | 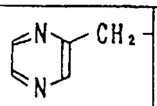 | H | $OCH_3$ | 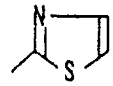 | |
| 391 | 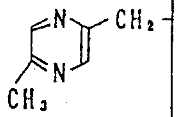 | 〃 | 〃 | 〃 | |
| 392 | 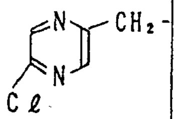 | 〃 | 〃 | 〃 | |
| 393 | 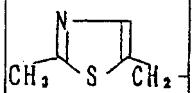 | 〃 | 〃 | 〃 | |
| 394 | 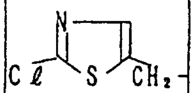 | 〃 | 〃 | 〃 | |
| 395 | 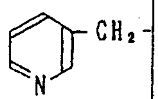 | 〃 | 〃 | 〃 | |
| 396 | 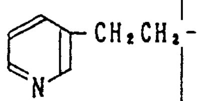 | 〃 | 〃 | 〃 | |
| 397 | 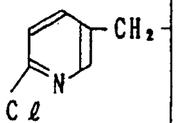 | 〃 | $SCH_3$ | 〃 | |
| 398 | 〃 | 〃 | $SC_2H_5$ | 〃 | |
| 399 | 〃 | 〃 | $SCH_2CH=CH_2$ | 〃 | |
| 400 | 〃 | 〃 | 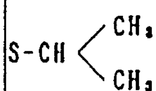 | 〃 | |
| 401 | 〃 | 〃 | $SCH_2C\equiv CH$ | 〃 | |
| 402 | 〃 | 〃 | 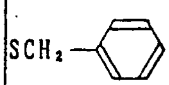 | 〃 | |
| 403 | 〃 | $CH_3$ | $SCH_3$ | 〃 | |
| 404 | 〃 | 〃 | $SC_2H_5$ | 〃 | |

| | | | | | |
|---|---|---|---|---|---|
| 405 | 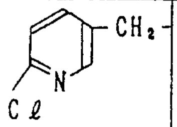 | 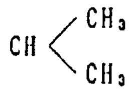 | $SCH_3$ | 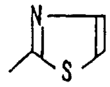 | |
| 406 | 〃 | 〃 | $SC_2H_5$ | 〃 | |
| 407 | 〃 | 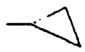 | $SCH_3$ | 〃 | |
| 408 | 〃 | 〃 | $SC_2H_5$ | 〃 | |
| 409 | 〃 | $CH_2CH=CH_2$ | $SCH_3$ | 〃 | |
| 410 | 〃 | 〃 | $SC_2H_5$ | 〃 | |
| 411 | 〃 | $CH_2C\equiv CH$ | $SCH_3$ | 〃 | |
| 412 | 〃 | 〃 | $SC_2H_5$ | 〃 | |
| 413 | 〃 | $COCH_3$ | $SCH_3$ | 〃 | |
| 414 | 〃 | $SO_2CH_3$ | 〃 | 〃 | |
| 415 | 〃 | H | 〃 | 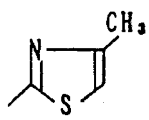 | [125-126] |
| 416 | 〃 | 〃 | 〃 | 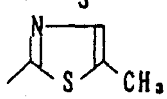 | [94-95] |
| 417 | 〃 | 〃 | 〃 | 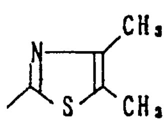 | [99-100] |
| 418 | 〃 | 〃 | 〃 | 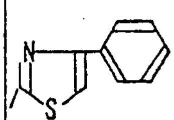 | [129-130] |
| 419 | 〃 | 〃 | 〃 | 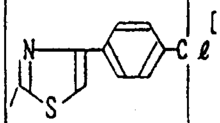 | [152-153] |

| | | | | | |
|---|---|---|---|---|---|
| 420 | 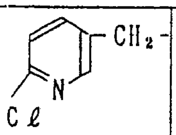 | H | $SCH_3$ | 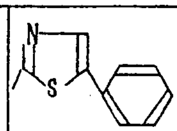 | [99-100] |
| 421 | 〃 | 〃 | 〃 | 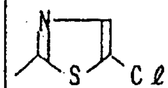 | [105-107] |
| 422 | 〃 | 〃 | 〃 | 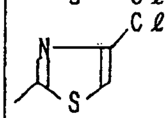 | |
| 423 | 〃 | 〃 | 〃 | 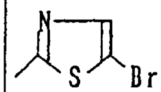 | |
| 424 | 〃 | 〃 | 〃 | 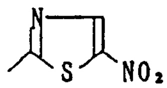 | [165-169] |
| 425 | 〃 | 〃 | 〃 | 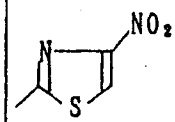 | |
| 426 | 〃 | 〃 | 〃 | 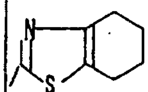 | |
| 427 | 〃 | 〃 | 〃 | 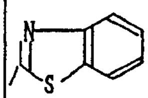 | |
| 428 | 〃 | 〃 | 〃 | 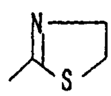 | |
| 429 | 〃 | 〃 | 〃 | 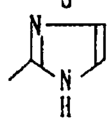 | [131-133] |
| 430 | 〃 | 〃 | 〃 | 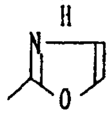 | |
| 431 | 〃 | 〃 | 〃 | 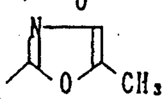 | |
| 432 | 〃 | 〃 | 〃 | 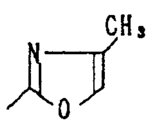 | |

| | | | | | |
|---|---|---|---|---|---|
| 433 | 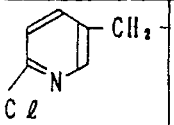 | H | $SCH_3$ | 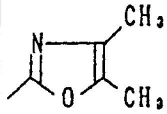 | |
| 434 | 〃 | 〃 | 〃 | 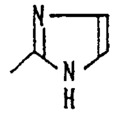 | |
| 435 | 〃 | 〃 | 〃 | 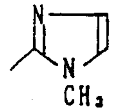 | |
| 436 | 〃 | 〃 | 〃 | 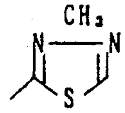 | |
| 437 | 〃 | 〃 | 〃 | 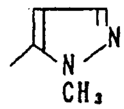 | |
| 438 | 〃 | 〃 | 〃 | 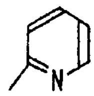 | |
| 439 | 〃 | 〃 | 〃 | 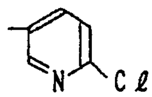 | [144-146] |
| 440 | 〃 | 〃 | 〃 | 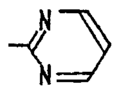 | $n_D^{25}$ 1.6417 |
| 441 | 〃 | 〃 | 〃 | 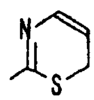 | |
| 442 | 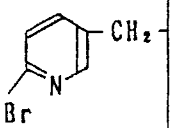 | 〃 | 〃 | 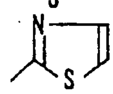 | |
| 443 | 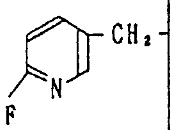 | 〃 | 〃 | 〃 | |
| 444 | 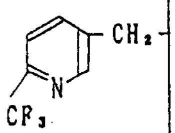 | 〃 | 〃 | 〃 | |
| 445 | 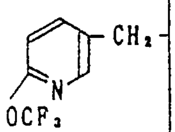 | 〃 | 〃 | 〃 | |

| | | | | | |
|---|---|---|---|---|---|
| 446 | 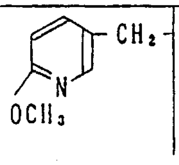 | H | $SCH_3$ | 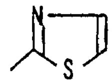 | |
| 447 | 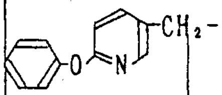 | 〃 | 〃 | 〃 | |
| 448 | 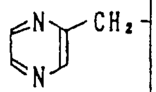 | 〃 | 〃 | 〃 | |
| 449 | 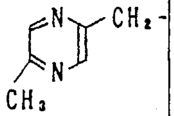 | 〃 | 〃 | 〃 | |
| 450 | 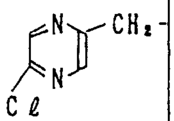 | 〃 | 〃 | 〃 | |
| 451 | 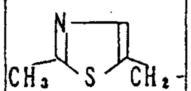 | 〃 | 〃 | 〃 | |
| 452 | 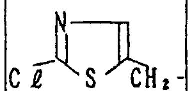 | 〃 | 〃 | 〃 | |
| 453 | 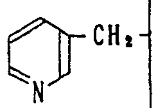 | 〃 | 〃 | 〃 | |
| 454 | 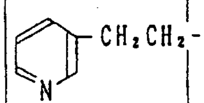 | 〃 | 〃 | 〃 | |
| 455 | 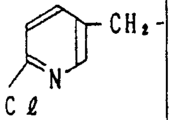 | $CH_2CH_2NH-$ | | 〃 | [134-135] |
| 456 | 〃 | 〃 | | 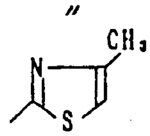 | |
| 457 | 〃 | 〃 | | 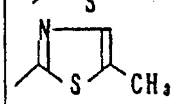 | |
| 458 | 〃 | 〃 | | 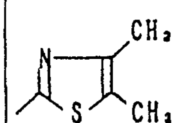 | |

| | | | | |
|---|---|---|---|---|
| 459 | 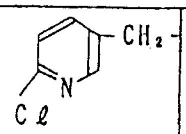 | $CH_2CH_2NH-$ | 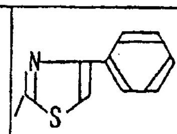 | |
| 460 | 〃 | 〃 | 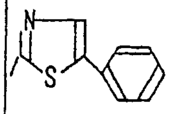 | |
| 461 | 〃 | 〃 | 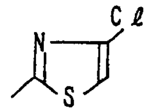 | |
| 462 | 〃 | 〃 | 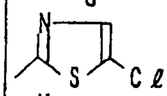 | |
| 463 | 〃 | 〃 | 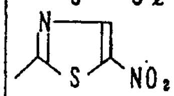 | |
| 464 | 〃 | 〃 | 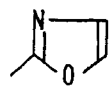 | |
| 465 | 〃 | 〃 | 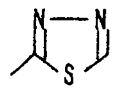 | |
| 466 | 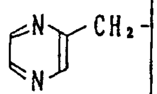 | 〃 | 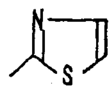 | $n_D^{25}$ 1.6517 |
| 467 | 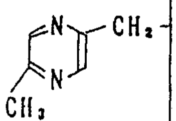 | 〃 | 〃 | [78-79] |
| 468 | 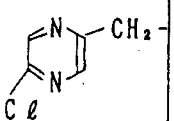 | 〃 | 〃 | |
| 469 | 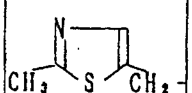 | 〃 | 〃 | |
| 470 | 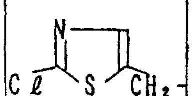 | 〃 | 〃 | $n_D^{25}$ 1.6562 |
| 471 | 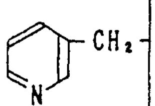 | 〃 | 〃 | |

| | | | | |
|---|---|---|---|---|
| 472 | 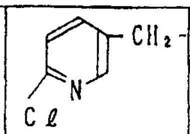 | $CH_2CH_2CH_2NH$ | 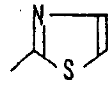 | $n_D^{25.5}$ 1.6531 |
| 473 | 〃 | 〃 | 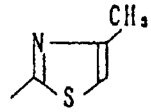 | |
| 474 | 〃 | 〃 | 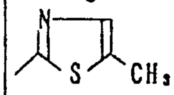 | |
| 475 | 〃 | 〃 | 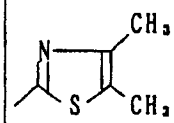 | |
| 476 | 〃 | 〃 | 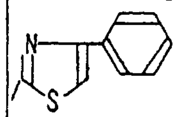 | |
| 477 | 〃 | 〃 | 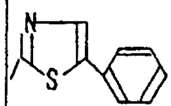 | |
| 478 | 〃 | 〃 | 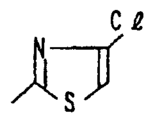 | |
| 479 | 〃 | 〃 | 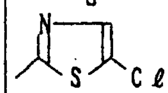 | |
| 480 | 〃 | 〃 | 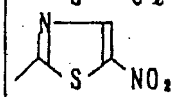 | |
| 481 | 〃 | 〃 | 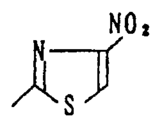 | |
| 482 | 〃 | 〃 | 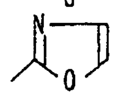 | |
| 483 | 〃 | 〃 | 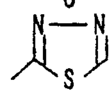 | |
| 484 | 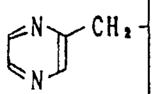 | 〃 | 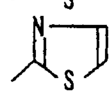 | |
| 485 | 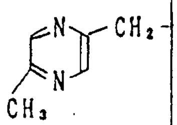 | 〃 | 〃 | |

| | | | | |
|---|---|---|---|---|
| 486 | 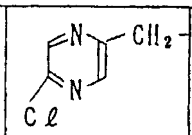 | $CH_2CH_2CH_2NH$ | 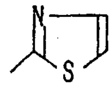 | |
| 487 | 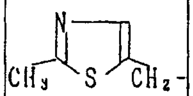 | 〃 | 〃 | |
| 488 | 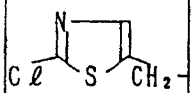 | 〃 | 〃 | |
| 489 | 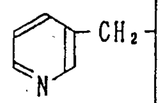 | 〃 | 〃 | |
| 490 | 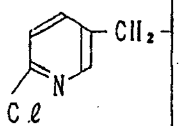 | $-CH_2CH_2S-$ | 〃 | [137-138] |
| 491 | 〃 | 〃 | 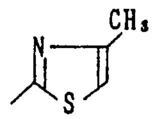 | |
| 492 | 〃 | 〃 | 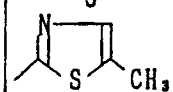 | |
| 493 | 〃 | 〃 | 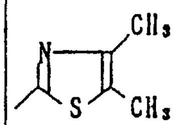 | |
| 494 | 〃 | 〃 | 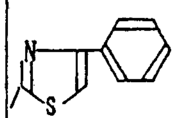 | |
| 495 | 〃 | 〃 | 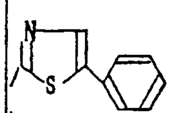 | |
| 496 | 〃 | 〃 | 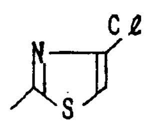 | |
| 497 | 〃 | 〃 | 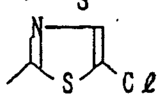 | |
| 498 | 〃 | 〃 | 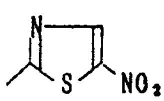 | |

| | | | | |
|---|---|---|---|---|
| 499 | 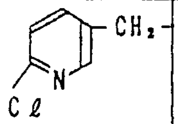 | $-CH_2CH_2S-$ | 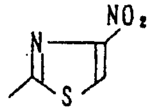 | |
| 500 | 〃 | 〃 | 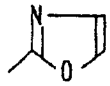 | |
| 501 | 〃 | 〃 | 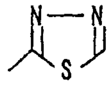 | |
| 502 | 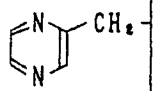 | 〃 | 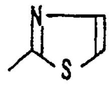 | |
| 503 | 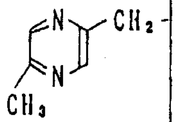 | 〃 | 〃 | |
| 504 | 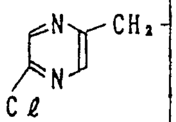 | 〃 | 〃 | |
| 505 | 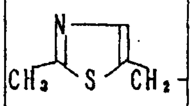 | 〃 | 〃 | |
| 506 | 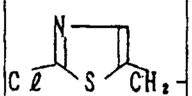 | 〃 | 〃 | |
| 507 | 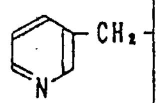 | 〃 | 〃 | |
| 508 | 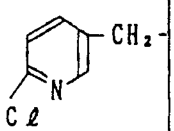 | $-CH_2CH_2CH_2S-$ | 〃 | |
| 509 | 〃 | 〃 | 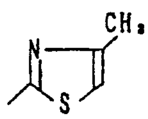 | |
| 510 | 〃 | 〃 | 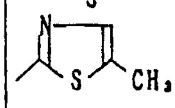 | |
| 511 | 〃 | 〃 | 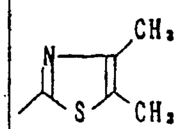 | |

| | | | | |
|---|---|---|---|---|
| 512 | 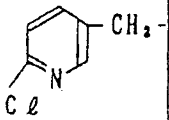 | $-CH_2CH_2CH_2S-$ | 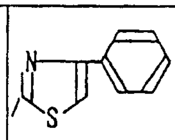 | |
| 513 | 〃 | 〃 | 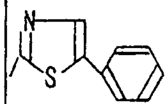 | |
| 514 | 〃 | 〃 | 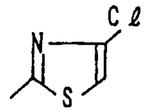 | |
| 515 | 〃 | 〃 | 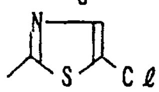 | |
| 516 | 〃 | 〃 | 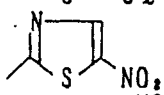 | |
| 517 | 〃 | 〃 | 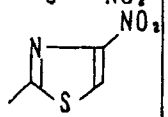 | |
| 518 | 〃 | 〃 | 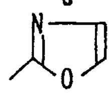 | |
| 519 | 〃 | 〃 | 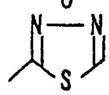 | |
| 520 | 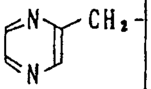 | 〃 | 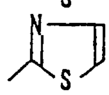 | |
| 521 | 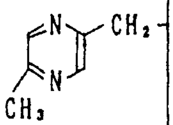 | 〃 | 〃 | |
| 522 | 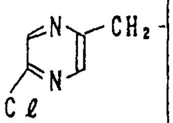 | 〃 | 〃 | |
| 523 | 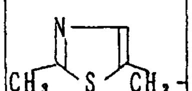 | 〃 | 〃 | |
| 524 | 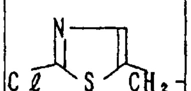 | 〃 | 〃 | |
| 525 | 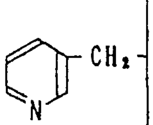 | 〃 | 〃 | |

| | | | | |
|---|---|---|---|---|
| 526 | 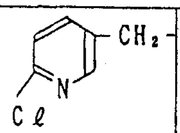 | $-CH_2CH_2O-$ | 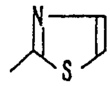 | |
| 527 | 〃 | 〃 | 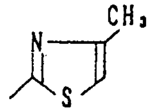 | |
| 528 | 〃 | 〃 | 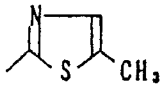 | |
| 529 | 〃 | 〃 | 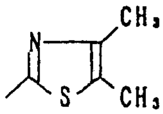 | |
| 530 | 〃 | 〃 | 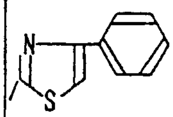 | |
| 531 | 〃 | 〃 | 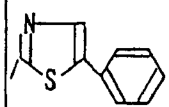 | |
| 532 | 〃 | 〃 | 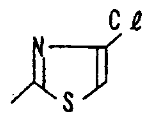 | |
| 533 | 〃 | 〃 | 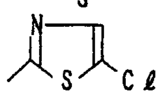 | |
| 534 | 〃 | 〃 | 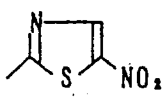 | |
| 535 | 〃 | 〃 | 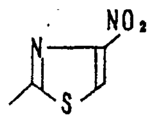 | |
| 536 | 〃 | 〃 | 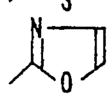 | |
| 537 | 〃 | 〃 | 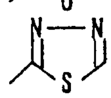 | |
| 538 | 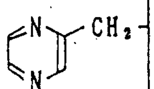 | 〃 | 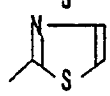 | |
| 539 | 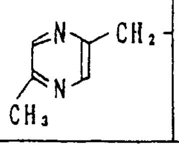 | 〃 | 〃 | |

| | | | | |
|---|---|---|---|---|
| 540 | 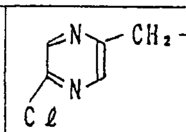 | $-CH_2CH_2O-$ | 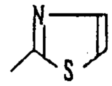 | |
| 541 | 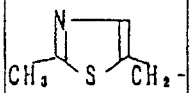 | ″ | ″ | |
| 542 | 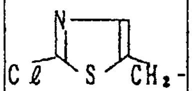 | ″ | ″ | |
| 543 | 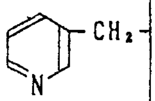 | ″ | ″ | |
| 544 | 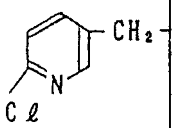 | $-CH_2CH_2CH_2O-$ | ″ | |
| 545 | ″ | ″ | 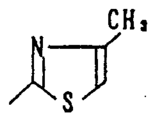 | |
| 546 | ″ | ″ | 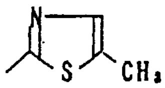 | |
| 547 | ″ | ″ | 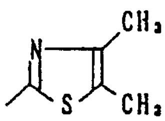 | |
| 548 | ″ | ″ | 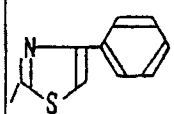 | |
| 549 | ″ | ″ | 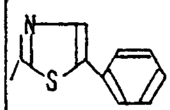 | |
| 550 | ″ | ″ | 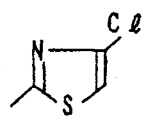 | |
| 551 | ″ | ″ | 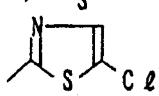 | |
| 552 | ″ | ″ | 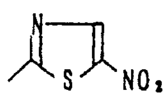 | |

| | | | | |
|---|---|---|---|---|
| 553 | 〃 | 〃 | 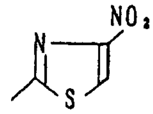 | |
| 554 | 〃 | 〃 | 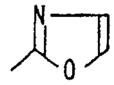 | |
| 555 | 〃 | 〃 | 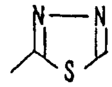 | |
| 556 | 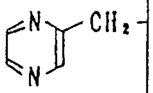 | 〃 | 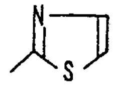 | |
| 557 | 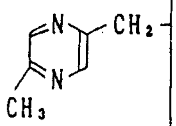 | 〃 | 〃 | |
| 558 | 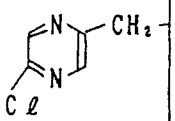 | 〃 | 〃 | |
| 559 | 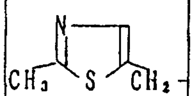 | 〃 | 〃 | |
| 560 | 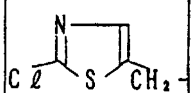 | 〃 | 〃 | |
| 561 | 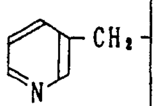 | 〃 | 〃 | |
| 562 | 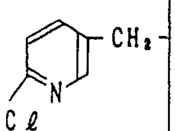 | $CH_2CH_2CH_2-$ | 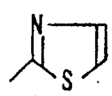 | |
| 563 | 〃 | 〃 | 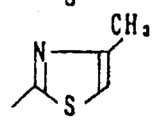 | |
| 564 | 〃 | 〃 | 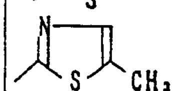 | |
| 565 | 〃 | 〃 | 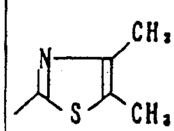 | |

| | | | | |
|---|---|---|---|---|
| 566 | 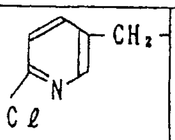 | $CH_2CH_2CH_2-$ | 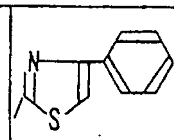 | |
| 567 | 〃 | 〃 | 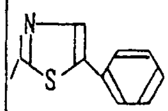 | |
| 568 | 〃 | 〃 | 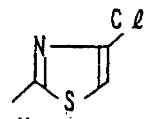 | |
| 569 | 〃 | 〃 | 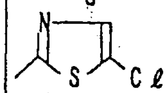 | |
| 570 | 〃 | 〃 | 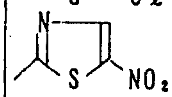 | |
| 571 | 〃 | 〃 | 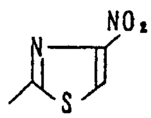 | |
| 572 | 〃 | 〃 | 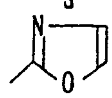 | |
| 573 | 〃 | 〃 | 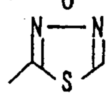 | |
| 574 | 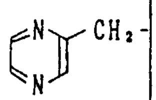 | 〃 | 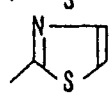 | |
| 575 | 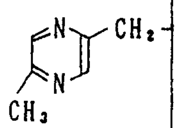 | 〃 | 〃 | |
| 576 | 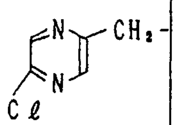 | 〃 | 〃 | |
| 577 | 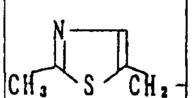 | 〃 | 〃 | |
| 578 | 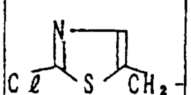 | 〃 | 〃 | |
| 579 | 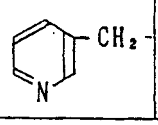 | 〃 | 〃 | |

| | | | | |
|---|---|---|---|---|
| 580 | 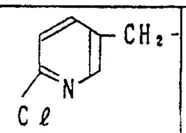 | $CH_2CH_2CH_2CH_2$ | 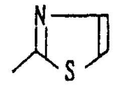 | |
| 581 | 〃 | 〃 | 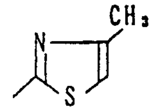 | |
| 582 | 〃 | 〃 | 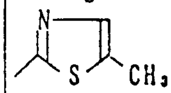 | |
| 583 | 〃 | 〃 | 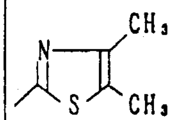 | |
| 584 | 〃 | 〃 | 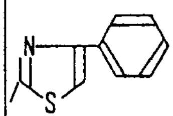 | |
| 585 | 〃 | 〃 | 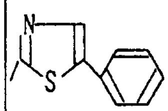 | |
| 586 | 〃 | 〃 | 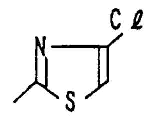 | |
| 587 | 〃 | 〃 | 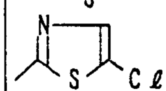 | |
| 588 | 〃 | 〃 | 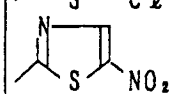 | |
| 589 | 〃 | 〃 | 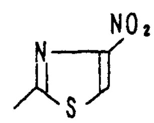 | |
| 590 | 〃 | 〃 | 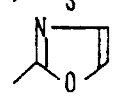 | |
| 591 | 〃 | 〃 | 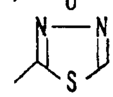 | |
| 592 | 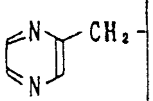 | 〃 | 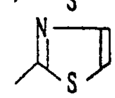 | |
| 593 | 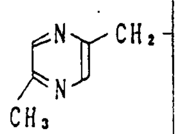 | 〃 | 〃 | |

| | | | | | |
|---|---|---|---|---|---|
| 594 | 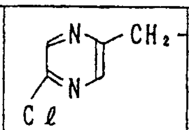 | $CH_2CH_2CH_2CH_2$ | | 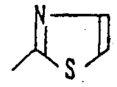 | |
| 595 | 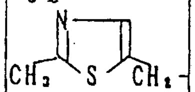 | 〃 | | 〃 | |
| 596 | 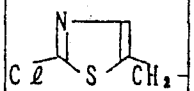 | 〃 | | 〃 | |
| 597 | 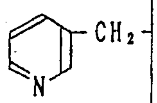 | 〃 | | 〃 | |
| 598 | 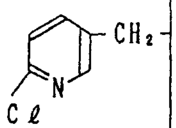 | $C_2H_5$ | $CH_3$ | 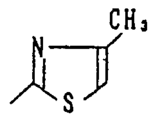 | [79-81] |
| 599 | 〃 | 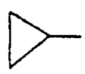 | 〃 | 〃 | [98-101] |
| 600 | 〃 | 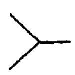 | 〃 | 〃 | [143-145] |
| 601 | 〃 | H | $C_2H_5$ | 〃 | $n_D^{25}$ 1.6130 |
| 602 | 〃 | $CH_3$ | 〃 | 〃 | $n_D^{25}$ 1.6092 |
| 603 | 〃 | H | $C_3H_7$(n) | 〃 | $n_D^{25}$ 1.6065 |
| 604 | 〃 | $CH_3$ | 〃 | 〃 | [77-80] |
| 605 | 〃 | H | $C_3H_7$(i) | 〃 | $n_D^{25}$ 1.5996 |
| 606 | 〃 | $CH_3$ | 〃 | 〃 | $n_D^{25}$ 1.5924 |
| 607 | 〃 | $OCH_3$ | $CH_3$ | 〃 | $n_D^{25}$ 1.5958 |

The compounds in the invention exhibit outstandingly insecticidal activity against many kinds of pest insects such as army worm, diamond moth, aphids, green rice leaf hopper, brown rice plant hopper and others.

Recently, resistant strain for organophosphate and carbamate insecticides has been developed in many kinds of pest insect such as diamond moth, plant hoppers, leaf hoppers and aphids, so the problems on lack of activity of the insecticides is resulted, contribution of newer effective insecticide against the resistant insects has been expected, The compounds in the invention have not only outstanding effectiveness against susceptible insects but also against the resistant strains to organophosphate and carbamate insecticides.

The insecticidal compositions in the invention contains the compounds represented general in formula [I] as an active ingredient, which is allowed to be applied as the pure state, but usually applied as commonly recommended pesticide formulations such as wettable powder, water soluble powder, dust, emulsifiable concentrate, granular and flowable. In the case of solid formulation desired powdery plant materials such as soybean powder and wheat flour, mineral fine powders such as diatomaceous earth, apatite, gypsum, talc, bentonite and clay, and inorganic substances such as sodium benzoate, urea and sodium sulfate are utilized as their carriers and additives.

In the case of the liquid formulations desired, vegetable oils (seed oil) mineral oils, distillate fractions of petroleum such as kerosene, xylene and solvent naphtha, and cyclohexane, cyclohexanone dimethylformamide, dimethylsulfoxide, trichloroethylene, methyl isobutyl ketone and water are utilized as the solvent. In order to keep homogeneous and stable dilution, if necessary, surface active agents are allowable to be added. Thus prepared wettable powder, emulsifiable concentrate, water soluble powder and flowable are diluted to an indicated concentration as suspension, emulsion in water or aqueous solution. Then, they are sprayed, but dust and granular are applied without any dilution, for crop plants.

Although the compounds in the invention are, of course, sufficiently effective even in single use, they are also allowable to be blended with many kinds of insecticides, acaricides and fungicides.

Insecticides, acaricides and fungicide with which the invented compounds are allowable to be blended are shown as follows:

As acaricides (fungicides), for example, chlorobenzilate, chloropropilate, proclonol, phenisobromolate, docofol, dinobuton. binapacryl, chlorophenamidin, amitoraz, BPPS, PPPS, benzomate, hexythiazox, phenbutatin oxide, polynactin, quinomethionate, thioquinox, CPCBS, tetradifon, kayacide, avermectin, calcium polysulfide, clofentezine, fulbenzimin, fulfenoxuron, BCPE, cyhexatin, pyridaben, fenpyroximate, thiophanate methyl, benomyl, thiuram, IBP, EDDP, fthalide, probenazole, isoprothiolane, TPN, captan, polyoxin, blastcidin-S, kasugamycin, Validamycin, tricyclazole, pyroquilon, phenazin oxide, mepronil, fultoranil, pencycuron, ipurodione, hymexazol, metalaxil, triflumizol, diclomezin and tecloftalam;

As organophosphate and carbamate insecticides (acaricides), for example, fenthion, fenitrothion, diazinon, chlorpyriphos, ESP, vamidothion, fenthoate, dimethoate, formothion, malathion, dipterex, thiomethon, phosmet, menazon, dichlorvos, acephate, EPBP, diaryfol, methyl parathion, oxydimethon methyl, ethion, pyrachlofos, monocrotophos, aldicarb, propoxur, methomyl, BPMC, MTMC, NAC, cartap, carbosulfan, benfuracarb pyrimicarb, ethofencarb, phenoxycarb and thiodicarb:

As pyrethroides insecticides (acaricides), for example, permethrin, cypermethrin, decamethrin, fenvalerate, fenpropathrin, pyrethrin, allethrin, tetramethrin, resmethrin, dimethrin propathrin, bifenthrin, prothrin, fluvalinate, cyfluthrin, cyhalothrin, flucythrinate, ethofenprox, cycloprothrin, tralomethrin, silaneophne:

As benzoylphenylureas and other insecticides, for example, diflubenzuron, chlofluazuron, triflumuron, teflubenzuron, buprofezin and machine oil.

The examples of the formulations are shown as follows: The scope of the present invention, however, shall not be limited by these descriptions.

Example 4

| Emulsifiable concentrate | |
|---|---|
| The invented compound | 10 parts |
| alkylphenylpolyoxyethylene | 5 parts |
| dimethylformamide | 50 parts |
| xylene | 35 parts |

These are mixed to be a solution and sprayed by dilution with water as it use.

Example 5

| Wettable powder | |
|---|---|
| The invented compound<br>sulfate ester of higher alcohol<br>diatomaceous earth<br>artificial silicate | 20 parts<br>5 parts<br>70 parts<br>5 parts |

These are mixed, micronized to be fine powder and sprayed as a suspension in water by dilution with as it use.

Example 6

| Dust formulation | |
|---|---|
| The invented compound<br>talc<br>artificial silicate | 5 parts<br>94.7 parts<br>0.3 parts |

These are mixed, pulverized to be powder and sprayed without any dilution as it use.

Example 7

| Granular formulation | |
|---|---|
| The invented compound<br>clay<br>bentonite<br>sodium dioctylsuccinate<br>sodium phosphate | 5 parts<br>73 parts<br>20 parts<br>1 part<br>1 part |

These are granulated and applied without any dilution.

Industrial Applicability

Test example 1. Control effect against cotton aphid

Cotton aphids (Aphis gassypii) of 30-50 were inoculated oil leaves of cucumber seedling at 10 days after the emergence by using a painting fine brush. One day after the inoculation the injured or damaged aphids were removed and then the diluted emulsion to be 125 ppm of active ingredient of the compounds formulated as emulsifiable concentrate according to the example 4 were sprayed. The pot was kept in a chamber at 25 C under 65 % of humidity for 7 days. The survival number of the aphids was counted and the insect control ratio was obtained by comparing with untreated number of the survivals. The results are shown in Table 2.

Table 2

| Compound No. | Control Efficacy (7days later) 125ppm |
|---|---|
| 2 | 100% |
| 19 | 100 |
| 20 | 100 |
| 58 | 100 |
| 59 | 100 |
| 60 | 100 |
| 61 | 100 |
| 62 | 100 |
| 63 | 100 |
| 64 | 100 |
| 65 | 100 |
| 66 | 100 |
| 67 | 100 |
| 68 | 100 |
| 69 | 100 |
| 101 | 100 |
| 109 | 100 |
| 143 | 100 |
| 160 | 100 |
| 161 | 100 |
| 175 | 100 |
| 226 | 100 |
| 229 | 100 |
| 230 | 100 |
| 232 | 100 |
| 234 | 100 |
| 236 | 100 |
| 238 | 100 |
| 240 | 100 |
| 246 | 100 |
| 415 | 100 |
| 416 | 100 |
| 421 | 100 |
| 455 | 100 |
| 467 | 100 |
| 470 | 100 |
| 472 | 100 |
| Comparative Compound A | 0 |

Comparative Compound A: Pyrimicarb

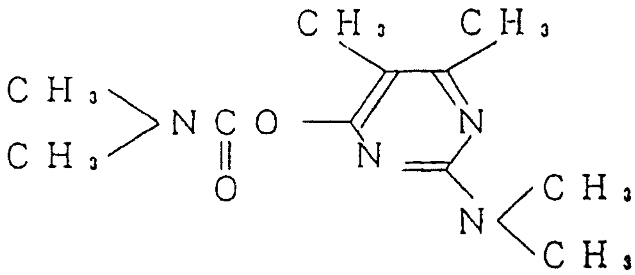

Test example 2. Control effect against green rice leaf hopper.

Infantile rice seedling at 7 days after the emergence was immersed with the diluted emulsion to be 125 ppm of active ingredient of the compounds similarly formulated emulsifiable concentrate as preceding "Test examples" for 30 seconds. After naturally drying the treated seedling was admitted in a test tube and 10 of third instar larvae of green rice leaf hopper (Nephotettix cincticeps) which was resistant strain for both organophosphorus and carbamate insecticides were inoculated. Covering by gauze, the test tube was kept in a chamber at 25°C under 65 % humidity for 5 days. Then the insecticidal activity was investigated. The results are shown in Table 3.

Table 3

| Compound No. | Mortality (5days later) 125ppm |
| --- | --- |
| 2 | 100% |
| 19 | 100 |
| 20 | 100 |
| 58 | 100 |
| 59 | 100 |
| 61 | 100 |
| 63 | 100 |
| 65 | 100 |
| 67 | 100 |
| 69 | 100 |
| 83 | 100 |
| 101 | 100 |
| 115 | 100 |
| 161 | 100 |
| 175 | 100 |
| 226 | 100 |
| 229 | 100 |
| 230 | 100 |
| 232 | 100 |
| 234 | 100 |
| 236 | 100 |
| 238 | 100 |
| 240 | 100 |
| 246 | 100 |
| 415 | 100 |
| 416 | 100 |
| 417 | 100 |
| 418 | 100 |
| 419 | 100 |
| 420 | 100 |
| 421 | 100 |
| 424 | 100 |
| 429 | 100 |
| 455 | 100 |
| 470 | 100 |
| 472 | 100 |
| Comparative Compound B | 0 |

Comparative Compound B: malathion

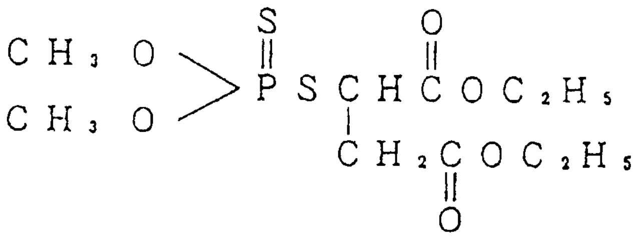

## Claims

1. The compounds which are represented by general formula [I] or their salts, the processes for preparing them and the insecticidal compositions containing them.

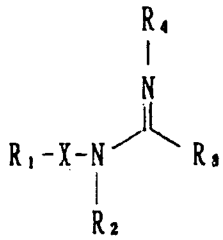

[I]

[wherein, $R_1$ is aromatic heterocycle containing nitrogen allowed to be substituted; X is alkylene or alkylidene allowed to be substituted in each case;

$R_2$ is hydrogen, alkyl, alkenyl, alkynyl, cycloalkyl, cycloalkenyl, aryl allowed to be substituted in each case or $-Y-R_5$, wherein Y is O, S(O)n, -CO- or $-CO_2-$, wherein n denotes 1 or 2 and $R_5$ is hydrogen, alkyl, alkenyl, alkynyl, cycloalkyl cycloalkenyl or aryl allowed to be substituted in each case;

$R_3$ is hydrogen, alkyl, alkenyl, alkynyl, cycloalkyl, cycloalkenyl, heterocycles allowed to be substituted, $OR_6$, $SR_6$ (wherein, $R_5$ is hydrogen, alkyl, alkenyl, alkynyl, cycloalkyl, cycloalkenyl or aryl allowed to be substituted in each case) or $-NR_7R_8$, (wherein $R_7$ and $R_8$ are individually hydrogen, alkyl, alkenyl, alkynyl, cycloalkyl, cycloalkenyl, aryl allowed to be substituted in each case or $Z-R_9$ wherein Z is O, $S(O)_k$ -CO or $CO_2$ in which k denotes zero, 1 or 2 and $R_9$ is hydrogen, alkyl, alkenyl, alkynyl, cycloalkyl, cycloalkenyl or aryl allowed to be substituted in each case), $R_7$ and $R_8$ and /or, $R_2$ and $R_3$ are allowed to form a ring by binding in each other which may contain further hetero atom; and $R_4$ is heterocycle containing nitrogen allowed to be substituted].

2. A process for preparing compounds represented by general formula [I], which comprises reacting compounds [II] and compounds [III].

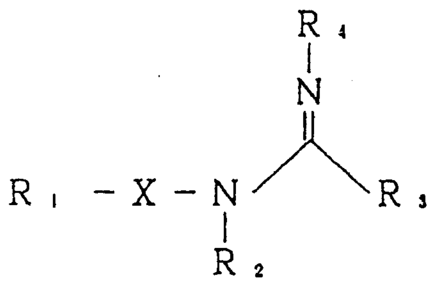

( I )

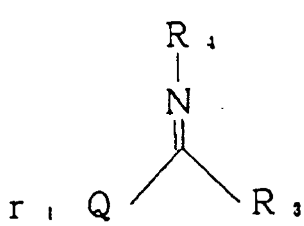

( II )

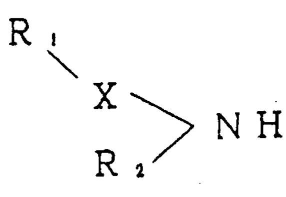

( III )

Wherein $r_1$ is lower alkyl; Q is oxygen or sulfar; and X, $R_1$ -$R_4$ are same as above.

**3.** A process for preparing compounds represented by general formula [I'], which comprises reacting compounds [IV] and compounds [V].

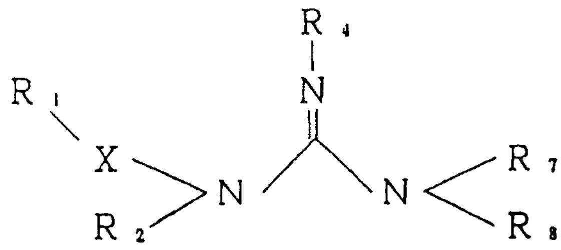

[I']

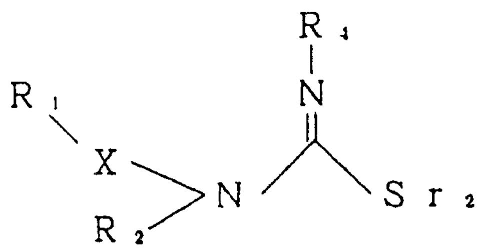

[IV]

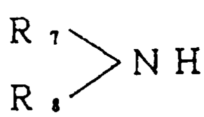

[V]

Wherein $r_2$ is lower alkyl; and X, $R_1$, $R_2$, $R_4$, $R_7$ and $R_8$ are same as above.

**4.** A process for preparing compounds represented by general formula [I''], which comprises reacting compounds [VI] and compounds [VII].

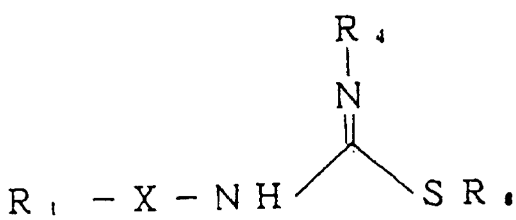

[I'']

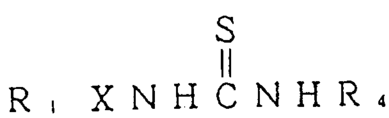

[VI]

$Hal-R_8$ [VII]

Wherein Hal is halogen; and X, $R_1$, $R_4$, and $R_8$ are same as above.

**5.** A process for preparing compounds represented by general formula [I'''], which comprises reacting compounds [VIII] and compounds [IX].

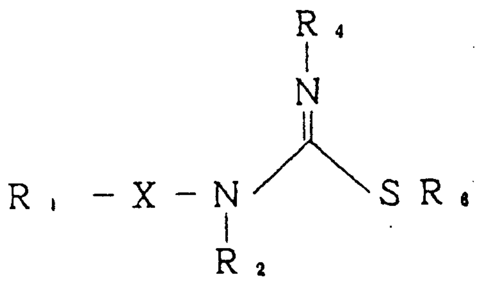

[I''']

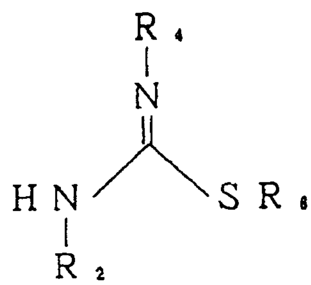

[VIII]

$R_1-X-Hal$ [IX]

Wherein Hal is halogen; and X, $R_1$, $R_2$, $R_4$ and $R_6$ are same as above.

**6.** A Process for preparing compounds represented by general formula [$I^{4}$'], which comprises reacting compounds [X] and compounds [XI].

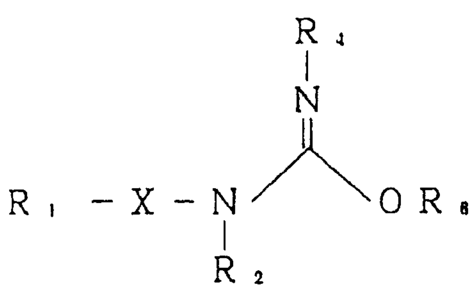

[$I^{4}$']

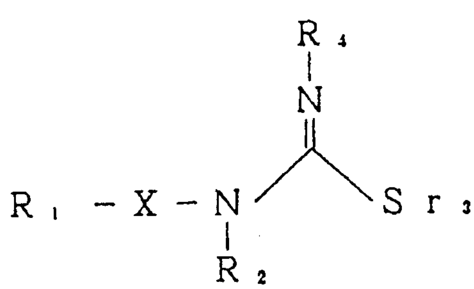

[X]

$R_6\,OH$ [XI]

Wherein $r_3$ is lower alkyl, and X, $R_1$, $R_2$, $R_4$ and $R_6$ are same as above.

**7.** A process for preparing compounds represented by general formula [$I^{5}$'], which comprises reacting compounds [XII] and compounds [XIV] or [XV].

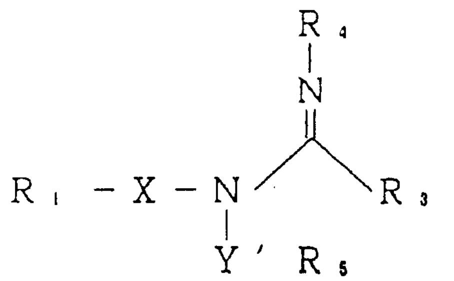

[I5']

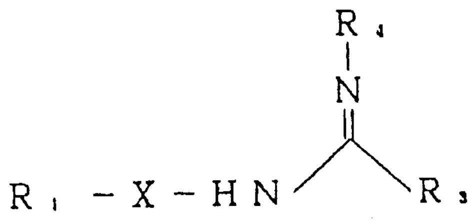

[XII]

$VY'R_5$ [XIV]

$(R_5CO)_2O$ [XV]

Wherein V is halogen; Y' is -S(O)n-, -CO- or $-CO_2-$; and n, X, $R_1$, $R_3$, $R_4$ and $R_5$ are same above.

**8.** A process for preparing compounds represented by by general formula [I6'], which comprises cyclizing compounds [XVI] by heating under presence of deacidifying agent.

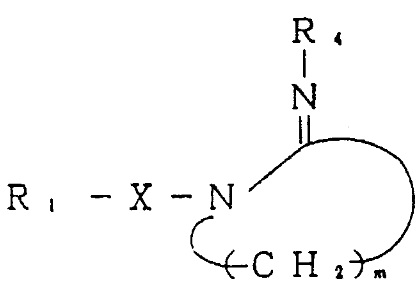

[I6']

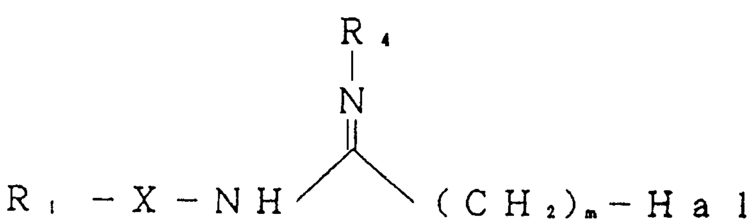

[XVI]

Wherein m is 2, 3 or 4; and Hal, X, $R_1$ or $R_4$ are same as above.

**9.** A process for preparing compounds represented by general formula [I7'], which comprises reacting compounds [XVII] and compounds [XVIII].

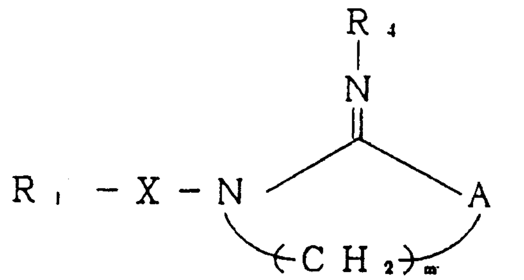

[I 7']

$R_1XNH-(CH_2)_m-AH$ [XVII]

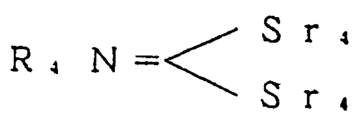

[XVIII]

Wherein A is O, S or NH; $r_4$ is lower alkyl; and m, X, $R_1$ and $R_4$ are same as above.

**10.** Insecticidal compositions which are characterized to contain one or more than two kinds of the compounds represented by general formula [I] as the active ingredients.

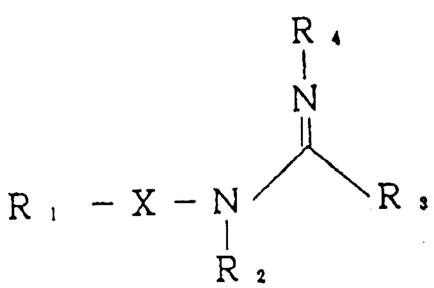

[I]

Wherein X, $R_1$-$R_4$ are same as above, and $r_4$ individually denote lower alkyl, Q denotes oxygen or sulfur atom, Hal and V denote halogen atom A denotes O, S or NH and Y' denotes -S(O)n-, -CO- or $-CO_2-$. The symbols, X, $R_1$, $R_2$, $R_3$, $R_4$, $R_5$, $R_8$ and n denote same as above described.

## Amended claims

**1.** The compounds which are represented by general formula [I] or their salts,

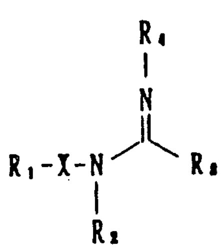

[I]

[wherein, $R_1$ is aromatic heterocycle containing nitrogen allowed to be substituted; X is alkylene or alkylidene allowed to be substituted in each case;

$R_2$ is hydrogen, alkyl, alkenyl, alkynyl, cycloalkyl, cycloalkenyl, aryl allowed to be substituted in each case or $-Y-R_5$, wherein Y is O, S(O)n, -CO- or $-CO_2-$, wherein n denotes 1 or 2 and $R_5$ is hydrogen, alkyl, alkenyl, alkynyl, cycloalkyl cycloalkenyl or aryl allowed to be substituted in each case;

$R_3$ is hydrogen, alkyl, alkenyl, alkynyl, cycloalkyl, cycloalkenyl, heterocycles allowed to be substituted. $OR_6$, $SR_6$ (wherein, $R_6$ is hydrogen, alkyl, alkenyl, alkynyl, cycloalkyl, cycloalkenyl or aryl allowed to be substituted in each case) or $-NR_7R_8$, (wherein $R_7$ and $R_8$ are individually hydrogen, alkyl, alkenyl, alkynyl, cycloalkyl, cycloalkenyl, aryl allowed to be substituted in each case or $Z-R_9$ wherein Z is O, $S(O)_k$ -CO or $CO_2$ in which k denotes zero, 1 or 2 and $R_9$ is hydrogen, alkyl, alkenyl, alkynyl, cycloalkyl, cycloalkenyl or aryl allowed to be substituted in each case), $R_7$ and $R_8$ and /or, $R_2$ and $R_3$ are allowed to form a ring by binding in each other which may contain further hetero atom; and $R_4$ is heterocycle containing nitrogen allowed to be substituted].

**2.** A process for preparing compounds represented by general formula [I], which comprises reacting compounds [II] and compounds [III].

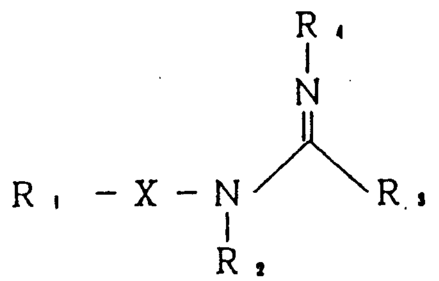

[ I ]

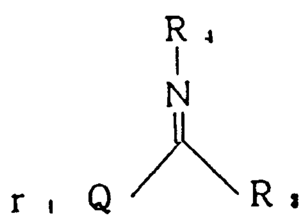

[ II ]

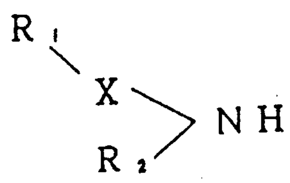

[ III ]

Wherein $r_1$ is lower alkyl; Q is oxygen or sulfur; and X, $R_1$-$R_4$ are the same as in claim 1.

**3.** A process for preparing compounds represented by general formula [I'], which comprises reacting compounds [IV] and compounds [V].

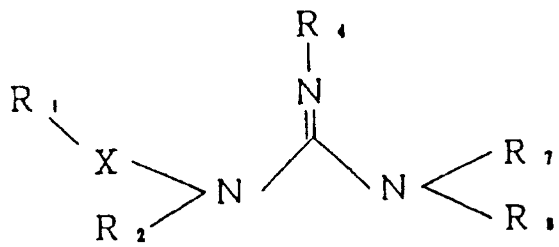

(I')

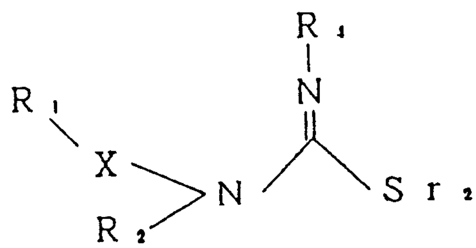

(IV)

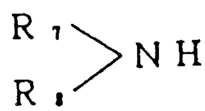

(V)

Wherein $r_2$ is lower alkyl; and X, $R_1$, $R_2$, $R_4$, $R_7$ and $R_8$ are the same as in claim 1.

**4.** A process for preparing compounds represented by general formula [I"] which comprises reacting compounds [VI] and compounds [VII].

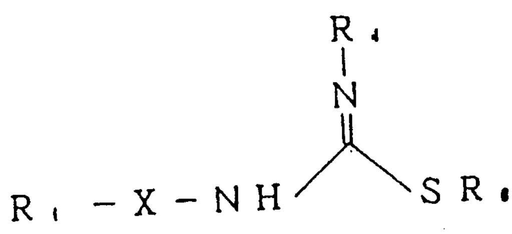

(I")

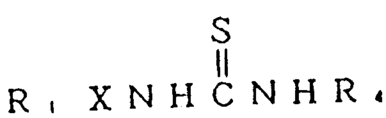

(VI)

$Hal-R_8$ (VII)

Wherein Hal is halogen; and X, $R_1$, $R_4$, and $R_8$ are same as in claim 1.

**5.** A process for preparing compounds represented by general formula [I'''], which comprises reacting compounds [VIII] and compounds [IX].

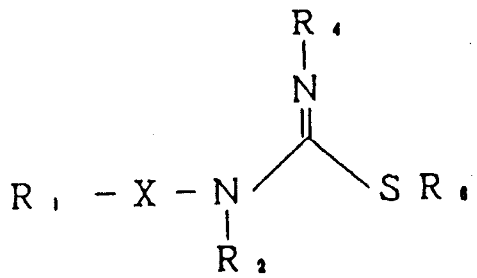

(I''')

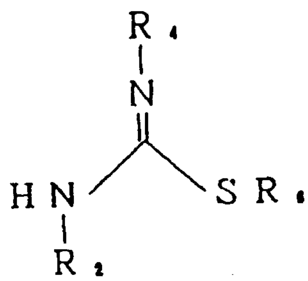

(VIII)

$$R_1-X-Hal$$ (IX)

Wherein Hal is halogen: and X, $R_1$, $R_2$, $R_4$ and $R_6$ are same as in claim 1.

**6.** A process for preparing compounds represented by general formula [I4'], which comprises reacting compounds [X] and compounds [XI].

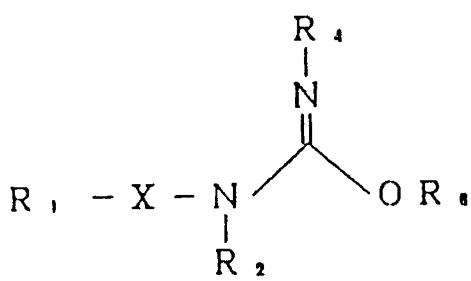

(I4')

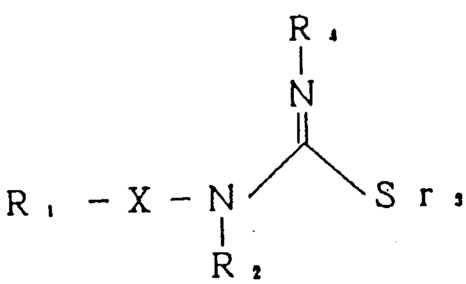

(X)

$$R_6OH$$ (XI)

Wherein $r_3$ is lower alkyl, and X, $R_1$, $R_2$, $R_4$ and $R_6$ are same as in claim 1.

**7.** A process for preparing compounds represented by general formula [I5'], which comprises reacting compounds [XII] and compounds [XIV] or [XV].

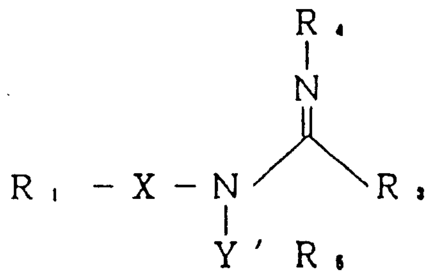

[I5']

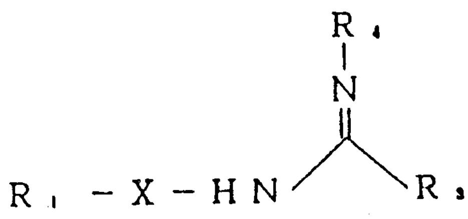

[XII]

$VY'R_5$ [XIV]

$(R_5CO)_2O$ [XV]

Wherein V is halogen; Y' is -S(O)n-, -CO- or $-CO_2-$; and n, X, $R_1$, $R_3$, $R_4$ and $R_5$ are the same as in claim 1.

**8.** A process for preparing compounds represented by general formula [$I^{6}$'], which comprises cyclizing compounds [XVI] by heating under presence of deacidifying agent.

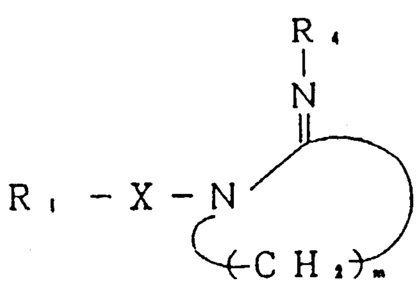

[I6']

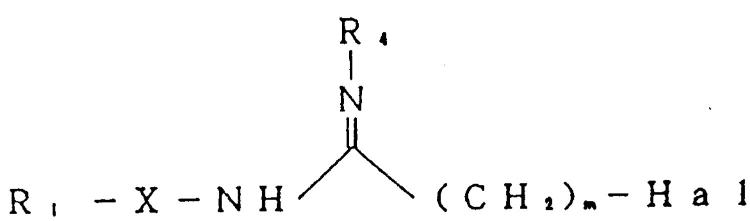

[XVI]

Wherein m is 2, 3 or 4; and Hal, X, $R_1$ or $R_4$ are same as in claim 1.

**9.** A process for preparing compounds represented by general formula [$I^{7}$'], which comprises reacting compounds [XVII] and compounds [XVIII].

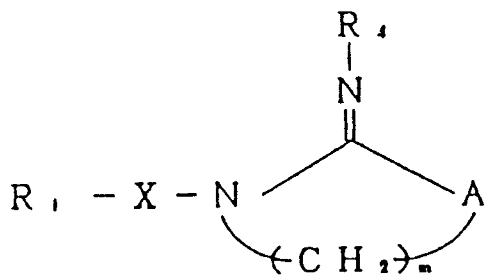

[I 7']

$$R_1XNH-(CH_2)_m-AH \qquad [XVII]$$

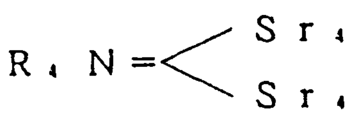

[XVIII]

Wherein A is O, S or NH; $r_4$ is lower alkyl; and m, X, $R_1$ and $R_4$ are the same as in claim 1.

**10.** Insecticidal compositions which are characterized to contain one or more of the compounds represented by general formula [I] as the active ingredients.

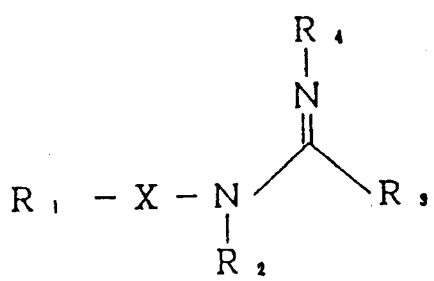

[I]

Wherein X, $R_1$-$R_4$ are the same as in claim 1.

# INTERNATIONAL SEARCH REPORT

International Application No PCT/JP91/00712

**I. CLASSIFICATION OF SUBJECT MATTER** (if several classification symbols apply, indicate all) [6]

According to International Patent Classification (IPC) or to both National Classification and IPC

Int. $Cl^5$ C07D213/77, C07D277/42, C07D401/12, C07D413/12, C07D417/12

**II. FIELDS SEARCHED**

Minimum Documentation Searched [7]

| Classification System | Classification Symbols |
|---|---|
| IPC | C07D213/77, C07D277/42, C07D401/12, C07D413/12, C07D417/12 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 61-12682 (Nippon Tokushu Noyaku Seizo K.K.), January 21, 1986 (21. 01. 86) & US, A, 4678795 & US, A, 4774247 & EP, A1, 163855 | 1-10 |
| Y | JP, A, 59-193878 (Fujisawa Pharmaceutical Co., Ltd.), November 2, 1984 (02. 11. 84) & US, A, 4649146 & EP, A2, 117082 & EP, A3, 117082 | 1-10 |
| Y | JP, A, 61-18775 (Bristol-Myers Co.), January 27, 1986 (27. 01. 86) & US, A, 4644006 & US, A, 4692531 & GB, A1, 2161157 | 1 |
| Y | JP, A, 63-270667 (Wakamoto Pharmaceutical Co., Ltd.), November 8, 1988 (08. 11. 88), (Family: none) | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 6, 1991 (06. 08. 91) | August 19, 1991 (19. 08. 91) |
| International Searching Authority | Signature of Authorized Officer |
| Japanese Patent Office | |